# EUROPEAN PATENT APPLICATION

(11) **EP 4 620 477 A1**
(43) Date of publication of application: **24.09.2025**
(21) Application number: 24305406.1
(22) Date of filing: 18.03.2024
(51) Int. Cl.: A61K 31/737, A61P 9/10, A61P 25/00

(54) **SYNTHETIC HEPARAN SULFATE BIOPOLYMERS FOR USE IN THE TREATMENT OF ISCHEMIC STROKE**

(71) Applicant: Organes Tissus Régénération Réparation Remplacement, 75001 Paris (FR); Barritault, Denis, 75001 Paris (FR)
(72) Inventor: BARRITAULT, Denis, 75001 Paris (FR); CHIAPPINI, Franck, 94200 Ivry-Sur-Seine (FR); CHOPPIN, Agnès, 78170 La Celle Saint Cloud (FR); INIZAN, Martin, 92000 Nanterre (FR); SEDEL, Frédéric, 75019 Paris (FR)
(74) Representative: Novagraaf Technologies

(57) **Abstract**

The present relates to a pharmaceutical composition for use in the treatment of ischemic stroke.

The present invention has an application in particular in the therapeutic and pharmaceutical fields.

## Description

### TECHNICAL FIELD

The present invention relates to a pharmaceutical composition for use in the treatment of ischemic stroke.

The present invention has an application in particular in the therapeutic, pharmaceutical and veterinary fields.

In the following description, the references between parentheses () refer to the list of references presented at the end of the text.

### STATE OF THE ART

In 1970, the World Health Organization defined stroke as 'rapidly developed clinical signs of focal (or global) disturbance of cerebral function, lasting more than 24 hours or leading to death, with no apparent cause other than of vascular origin'. The Stroke Council of the American Heart Association/American Stroke Association in 2009 defined transient ischaemic attack: 'a transient episode of neurological dysfunction caused by focal brain, spinal cord or retinal ischaemia without acute infarction'. In 2013, the American Heart Association/American Stroke Association updated their endorsed definition of stroke to one that includes silent infarctions (inclusive of cerebral, spinal and retinal) and silent haemorrhages diagnosed by brain imaging techniques. The 'traditional' clinical definition of stroke is still included by the American Heart Association/American Stroke Association (Coupland et al., 2017 [5]).

Stroke affects 17 million people worldwide each year and is the second most common cause of death, causing 6.2 million deaths worldwide each year (Benjamin et al., 2018 [2], The Stroke Association. (2018)). A stroke occurs due to the disruption of blood flow to the brain by a bleed (hemorrhagic stroke) or a blockage (ischemic stroke), accounting for 15% and 85% of all strokes respectively. Ischemic stroke is caused by a focal occlusion or stenosis of an artery or multiple intracranial or extracranial cerebral arteries. There are four main ischemic stroke etiologies: 20% cardioembolic, 20% atherosclerosis (large artery disease), 25% lacunar (small vessel disease) and 5% other causes. Additionally, 30% are termed cryptogenic strokes, which includes strokes from unknown causes (Harpaz et al., 2020 [13]). There are various classification systems for acute ischemic stroke. The Oxford Community Stroke Project classification (OCSP, also known as the Bamford or Oxford classification) relies primarily on the initial symptoms; based on the extent of the symptoms, the stroke episode is classified as total anterior circulation infarct (TACI), partial anterior circulation infarct (PACI), lacunar infarct (LACI) or posterior circulation infarct (POCI). The TOAST (Trial of Org 10172 in Acute Stroke Treatment) classification is based on clinical symptoms as well as results of further investigations; on this basis, stroke is classified as being due to (1) thrombosis or embolism due to atherosclerosis of a large artery, (2) an embolism originating in the heart, (3) complete blockage of a small blood vessel, (4) other determined cause, (5) undetermined cause (two possible causes, no cause identified, or incomplete investigation).

In the core area of an acute ischemic stroke (AIS), blood flow is so drastically reduced that cells usually cannot recover and subsequently undergo cellular death. The tissue in the region bordering the infarct core, known as the ischemic penumbra, is less severely affected. This region is rendered functionally silent by reduced blood flow but remains metabolically active. Cells in this area are endangered but not yet irreversibly damaged. They may undergo cell death after several hours or days but if blood flow and oxygen delivery is restored shortly after the onset of stroke, they are potentially recoverable (Shi et al., 2018 [17]).

The management of acute ischemic stroke (AIS) has been significantly developed over the last two decades with the development of stroke units and progression of reperfusion therapies that target the salvage of the "ischemic penumbra"(refs in Shi et al., 2018 [17]; Guo & Miao, 2021 [11]).

Acute ischemic stroke is a medical emergency and during the acute phase, the treatment objective is the restauration of blood flow and the prevention of subsequent clots. The benefit of recanalization therapies in patients with AIS is strongly time-dependent, with earlier intervention achieving better outcomes (Guo & Miao, 2021 [11]).

Reperfusion strategies include": intravenous thrombolysis (IVT), and/or endovascular thrombectomy (EVT) to physically remove the blood clot. An EVT has to be carried out within hours of stroke onset and it can be performed as a complement to IV thrombolysis for large vessel occlusion only (Campbell et al., 2016 [3]). Ultimately, long-term rehabilitation therapy is available.

Despite improvements in reperfusion strategies, stroke is a leading cause of adult disability and stroke survivors can experience a wide range of long-lasting outcomes including problems with mobility, vision, speech, memory, personality changes, fatigue, epilepsy, and depression. The stroke-related illness, disability and early death set to double by 2035 (Feigin et al., 2014 [9]). Improved survival rates from stroke mean there will be more people living with stroke as a long-term condition.

There is therefore a real need in the state of the art for a compound and/or a composition that makes it possible to improve the treatment of ischemic stroke, in particular acute ischemic stroke, for example that could improve the ability and/or reduce the morbidity and/or the mortality.

After ischemic stroke, spontaneous tissue repair is generally limited (Cramer et al., 2008 [3]). Both endogenous and exogenous neuroprotective/neurotrophic substances may, nevertheless and to a variable extent, prevent neurons from degeneration and/or enable regeneration (Gutierrez-Fernandez et al., 2012 [12]). However, enzymatic degradation of these neuroprotective/neurotrophic substances, which occur rapidly after their release may limit their effects. The astrocytic scar limits secondary damage to brain tissue surrounding the ischemic area by restricting propagating cell death and by promoting chondroitin sulfate proteoglycan (CSPG)-mediated cell adhesion limiting cell migration as well. The formation of glial scars leads to increased neuronal death, demyelination and worsens neurological recovery in traumatic or ischemic brain injury (Dzyubenko et al., 2018 [8]). Therefore, the scar tissue is not usually constituted of new neurons, but mainly of glial cells.

There is therefore a real need in the state of the art for a compound and/or a composition that makes it possible to improve the treatment of ischemic stroke, in particular acute ischemic stroke, for example that could decrease neuronal death, demyelination and improve the neurological recovery in ischemic brain injury.

In the last decades, acute treatments aiming to recanalize the occluded vessel demonstrated efficacy in reducing the functional burden of the disease; however, reperfusion of the ischemic tissue brings some risk, with the most feared being hemorrhagic transformation (HT). HT is a common phenomenon after brain ischemia, occurs in up to 40% of patients treated with acute stroke therapy, and is fatal in around 3% of patients (Arba et al., 2021 [1]). In vitro and in vivo models suggested failure of endothelial integrity and loss of neurovascular homeostasis as the cellular mechanisms underlying blood extravasation and, from a structural point of view, disruption of the blood-brain barrier (BBB) as the pathophysiological step that leads to HT (Arba et al., 2021 [1]).

There is therefore a real need in the state of the art for a compound and/or a composition that makes it possible to decrease the risk of hemorrhagic transformation, in particular after reperfusion strategies.

It is also known in the art that with an aging population and access of developing countries to western lifestyle, the clinical and socioeconomic impact of stroke and stroke-related complications is on the rise.

Accordingly, there is therefore a real need in the state of the art for a compound and/or a composition that makes it possible to improve the treatment of ischemic stroke, in particular acute ischemic stroke, that would be more cost effective.

It is also known in the art that with the known treatment of ischemic stroke the rates of functional independence (modified Rankin scale 0-1) achieved even after highly effective reperfusion treatments are just below 50%, underscoring an unmet clinical need for adjunctive neuroprotective treatments (Goyal et al., 2016 [10], Cramer, 2015 [6]; Matei & Zang, 2021 [14]).

Accordingly, there is therefore a real need in the state of the art for a compound and/or a composition that makes it possible to improve the treatment of ischemic stroke, in particular acute ischemic stroke, that have better treatment results, in particular that could improve the rates of functional independence after treatment.

### DESCRIPTION OF THE INVENTION

The present invention aims specifically to address these needs by providing a pharmaceutical composition for use in the treatment of ischemic stroke, said composition comprising
- a biocompatible polymer of the following general formula (I)

   AaXxYy (I)

   wherein:
   A is a monomer,
   X is an R₁COOR₂ or -R₉(C=O)R₁₀ group,
   Y is an O or N-sulfonate group and has one of the following formulas -R₃OSO₃R₄, -R₅NSO₃R₆, R₇SO₃R₈ wherein:
   R₁, R₃, R₅ and R₉ are independently an aliphatic hydrocarbon chain, optionally branched and/or unsaturated and optionally containing one or more aromatic rings with the exception of benzylamine and benzylamine sulfonate, R₂, R₄, R₆ and R₈ are independently a hydrogen atom or an M⁺ cation,
   R₇ and R₁₀ are independently a bond, an aliphatic hydrocarbon chain, optionally branched and/or unsaturated,
   a is the number of monomers,
   x is the rate of substitution of the A monomers by X groups,
   y is the rate of substitution of the A monomers by Y groups
wherein said biocompatible polymer is administered in the treatment of ischemic stroke at a dose from 1 to 5 mg per day.

The present invention also provides a pharmaceutical composition for use in the treatment of ischemic stroke, said composition comprising
- a biocompatible polymer of the following general formula (I)

   AaXxYy (I)

   wherein:
   A is a monomer,
   X is an R₁COOR₂ or -R₉(C=O)R₁₀ group,
   Y is an O or N-sulfonate group and has one of the following formulas -R₃OSO₃R₄, -R₅NSO₃R₆, R₇SO₃R₈ wherein:
      R₁, R₃, R₅ and R₉ are independently an aliphatic hydrocarbon chain, optionally branched and/or unsaturated and optionally containing one or more aromatic rings with the exception of benzylamine and benzylamine sulfonate, R₂, R₄, R₆ and R₈ are independently a hydrogen atom or an M⁺ cation,
      R₇ and R₁₀ are independently a bond, an aliphatic hydrocarbon chain, optionally branched and/or unsaturated,
      a is the number of monomers,
      x is the rate of substitution of the A monomers by X groups,
      y is the rate of substitution of the A monomers by Y groups
wherein said biocompatible polymer is administered in the treatment of ischemic stroke at a dose of at least 10 µg/ml of initial stroke lesion volume.

Advantageously, the inventor has demonstrated surprisingly that the use of biocompatible polymer according to the invention, in particular the composition comprising a biocompatible polymer according to the invention makes it possible advantageously to treat ischemic stroke and the biological drawbacks of ischemic stroke.

The inventor has also surprisingly demonstrated that the use of biocompatible polymer according to the invention, and at a particular doses, allows advantageously to improve the clinical and MRI recovery of a patient after an ischemic stroke. The inventors have also demonstrated that the use of the biocompatible polymer according to the invention allows surprisingly to decrease the severity of hemorrhagic transformation that follows reperfusion. In others words, the inventors have also demonstrated that the use of the polymer according to the invention allows to decrease the risk of hemorrhagic transformation, in particular after reperfusion strategies. The inventors have further demonstrated that the use of biocompatible polymer allows to improve and accelerate the functional recovery, for example NIHSS and mRS scores of neurological dysfunction, of a patient after an ischemic stroke.

The inventor has also surprisingly demonstrated that the use of biocompatible polymer according to the invention, at particular doses, further allows advantageously to treat of intracerebral hemorrhage due to ischemic stroke.

The inventor has also surprisingly demonstrated that the use of biocompatible polymer according to the invention, at particular doses, further allows advantageously to prevent and/or treat of intracerebral hemorrhage due to reperfusion of ischemic stroke.

The inventor has also surprisingly demonstrated that the use of biocompatible polymer according to the invention, at particular doses, further allows to prevent and/or reduce hemorrhagic transformation due to reperfusion after ischemic stroke.

In the present, the terms "treatment", "cure", "treated" or "treat" refer to prophylaxis and/or therapy, in particular when the aim is to prevent and/or slow down (reduce) ischemic stroke, for example prevent and/or slow down (reduce) stroke lesion volume, necrotic infarct, ischemic core or hemorrhagic transformation or to accelerate clinical or stroke lesion volume recovery. Beneficial or desired clinical outcomes comprise, but are not limited to, alleviation of symptoms, acceleration of recovery, prevention of hemorrhagic transformation, improvement or palliation of the disease state, whether detectable or not. "Treatment" can also mean prolongation of survival and/or improvement in the quality of life compared to the survival and/or the quality of life expected if the patient does not receive treatment. Treatment can also mean decreased occurrence of adverse events such as intracranial hemorrhages. Advantageously, the treatment may comprise one of the following: reduction of the sensorimotor deficits, reduction of stroke lesion volume, reduction of necrotic infarct volume, reduction of ischemic core volume, improvement of sensorimotor recovery, reduction in the volume of the ischemic brain lesions, reduction of intracranial haemorrhages.

In the present, monomer means for example a monomer selected from the group consisting of sugars, esters, alcohols, amino acids or nucleotides or derivatives thereof.

In the present invention, the A monomers constitute the basic elements of the polymers of formula I and can be identical or different.

In the present invention, the identical or different A monomers can be independently selected from sugars or derivatives thereof.

In the present invention, the A monomers can be independently monomers of the following formula: 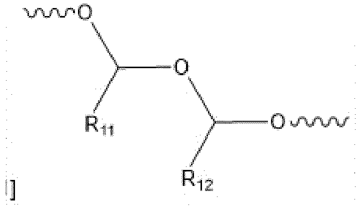 wherein R₁₁ and R₁₂ are independently an oxygen atom, an aliphatic hydrocarbon chain, optionally branched and/or unsaturated, a heteroaryl group comprising independently one or more oxygen and/or nitrogen atoms, an aldehyde function, a carboxylic acid group, a diol, a substituted diol, a group of formula -R₁₃-(X)n-R₁₄ wherein R₁₃ is a C₁ to C₄ aliphatic carbon chain, optionally branched and/or unsaturated, X is a heteroatom selected from oxygen and nitrogen, is an integer ranging from 1 to 4 and R₁₄ is a hydrogen atom, an aliphatic hydrocarbon chain, optionally branched and/or unsaturated, a heteroaryl group comprising independently one or more oxygen and/or nitrogen atoms, an aldehyde function, a carboxylic acid group, a diol, a substituted diol.

In the present invention, the combination of monomers can make it possible to form a polymeric backbone, for example a polymeric backbone of polyester, polyalcohol, polysaccharide, of the nucleic acid or protein type.

In the present invention, among the polyesters, these can be for example copolymers from biosynthesis or chemical synthesis, for example aliphatic polyesters or polyesters of natural origin for example polyhydroxyalkanoates.

In the present invention, the polysaccharides and their derivatives can be of bacterial, fungal, animal and/or plant origin. They can be for example single-chain polysaccharides, for example polyglucoses, for example dextran, cellulose, beta glucan, amidon/glycogen or other monomers comprising more complex units, for example xanthans, for example glucose, mannose, galactose, uronic acid and glucuronic acid, or glycuronans and glucoglucuronan, galactosamines, iduronic acid and/or protein derivatives highly glycosylated, for example mucins.

In the present invention, the polysaccharides of plant origin can be single-chain, for example cellulose (glucose), hemicellulose, pectins (galacturonic acid), fucans, starch or more complex like alginates (galuronic and mannuronic acid).

In the present invention, the polysaccharides of fungal origin can be for example steroglucan.

In the present invention, the polysaccharides of animal origin can be for example chitins or chitosan (glucosamine).

In the present invention, the A monomers that constitute the basic elements of the polymers of formula I can be advantageously identical.

In the present invention, the A monomers that constitute the basic elements of the polymers of formula I can be advantageously glucose.

The number of A monomers defined in formula (I) by "a" can be such that the weight of said polymers of formula (I) is greater than or equal to 1000 Daltons. The number of A monomers defined in formula (I) by "a" can be such that the weight of said polymers of formula (I) is between around 1 000 and 6000 Daltons, for example which corresponds to at least 5 glucose monomers. For example, the weight of said polymers of formula (I) can be between around 3000 Daltons and 6000 Daltons, for example which corresponds to 12 to 20 glucose monomers.

The number of A monomers defined in formula (I) by "a" can also be such that the weight of said polymers of formula (I) is less than about 2500000 Daltons (which corresponds to 7000 glucose monomers).

The number of A monomers defined in formula (I) by "a" can also be such that the weight of said polymers of formula (I) can be between around 2000 and 500000 Daltons, for example between 3000 and 500000 Daltons, for example equal to 3000 Daltons, 5000 Daltons, 6000 Daltons, 10000 Daltons, 20000 Daltons, 40000 Daltons, 80000 Daltons, 220000 Daltons, 500000 Daltons.

Advantageously, the weight of said polymers of formula (I) can be comprised from 3000 to 250000 Daltons, for example from 3000 to 6000 Daltons, or for example from 20000 to 250000 Daltons, or for example from 75000 to 150000 Daltons.

Advantageously, the weight of said polymers of formula (I) can be comprised from 3000 to 500000 Daltons, for example from 3000 to 250000 Daltons, for example from 3000 to 6000 Daltons, or for example from 20000 to 250000 Daltons, or for example from 75000 to 150000 Daltons.

In the present invention, in the -R₁COOR₂ group representing X, R₁ can be a C₁ to C₆ alkyl, for example a methyl, ethyl, butyl, propyl, pentyl, preferably a methyl group, and R₂ can be a bond, a C₁ to C₆ alkyl, for example a methyl, ethyl, butyl, propyl, pentyl, an R₂₁R₂₂ group wherein R₂₁ is an anion and R₂₂ a cation selected from the group of the alkali metals.

Preferably, the X group is the group of formula -R₁COOR₂ wherein R₁ is a methyl group -CH₂- and R₂ an R₂₁R₂₂ group wherein R₂₁ is an anion and R₂₂ a cation selected from the group of the alkali metals, preferably the X group is a group of formula -CH₂-COO⁻ or carboxymethyl.

In the present invention, in the -R₉(C=O)R₁₀ group representing X, R₉ can be a C₁ to C₆ alkyl, for example a methyl, ethyl, butyl, propyl, pentyl, preferably a methyl group, and R₁₀ can be a bond, a C₁ to C₆ alkyl, for example a methyl, ethyl, butyl, propyl, pentyl, hexyl.

The rate of substitution of all the A monomers by the X groups defined in the general formula (I) by "x" can be from 10 to 150%, from 40 to 80%, and preferably of the order of 50% or 60%.

In the present invention, in the group having one of the following formulas -R₃OSO₃R₄, -R₅NSO₃R₆, -R₇SO₃R₈ and representing the Y group, R₃ can be a bond, a C₁ to C₆ alkyl, for example a methyl, ethyl, butyl, propyl, pentyl, preferably a methyl group, R₅ can be a bond, a C₁ to C₆ alkyl, for example a methyl, ethyl, butyl, propyl, pentyl, preferably a methyl group, R₇ can be a bond, a C₁ to C₆ alkyl, for example a methyl, ethyl, butyl, propyl, pentyl, preferably a methyl group, R₄, R₆ and R₈ can be independently a hydrogen atom or a cation M⁺, for example M⁺ can be an alkali metal.

Preferably, the Y group is the group of formula -R₇SO₃R₈ wherein R₇ is a bond and R₈ is an alkali metal selected from the group comprising lithium, sodium, potassium, rubidium and cesium. Preferably, the Y group is a -SO₃⁻, - SO₃⁻ Na⁺ group

The rate of substitution of all the A monomers by the Y groups defined in the general formula (I) by "y" can be from 10 to 170%, from 30 to 150%, from 55 to 160%, from 55 to 85%, from 120 to 160%, and preferably of the order of 70, 140 or 150%.

In the present invention, the definition of the rates of substitution above, a rate of substitution "x" of 100% means that each A monomer of the polymer of the invention statistically contains an X group. Likewise, a rate of substitution "y" of 100% means that each monomer of the polymer of the invention statistically contains a Y group. Rates of substitution greater than 100% reflect the fact that each monomer statistically has more than one group of the considered type; conversely, rates of substitution of less than 100% reflect the fact that each monomer statistically has less than one group of the considered type.

The polymers can also comprise functional chemical groups, denoted Z, other than X and Y

In the present invention, the Z groups can be identical or different, and can be independently selected from the group comprising amino acids, fatty acids fatty alcohols, ceramides or derivatives thereof, nucleotide addressing sequences, antibodies, antibody fragments.

The Z groups can also be identical or different active agents. They can be, for example, therapeutic agents, diagnostic agents, an anti-inflammatory, an antimicrobial, an antibiotic, an antiviral agent, a growth factor, a cellular communication cytokine, for example an interferon, an enzyme, an antioxidant compound, polyphenols, tannins, anthocyanins, lycopenes, terpenoids and stilbenes for example resveratrol. In the present invention, the Z group can advantageously be a saturated or unsaturated fatty acid or cholesterol or derivative thereof. It can be a cholesterol derivative selected from the group comprising cholecalciferol, cortisone, cortisol, sex hormone or derivatives thereof. It can be for example a fatty acid selected from the group comprising acetic acid, caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, trans-vaccenic acid, linoleic acid, linolelaidic acid, α-linolenic acid, γ-linolenic acid, dihomo-γ-linolenic acid, arachidonic acid, eicosapentaenoic acid, clupanodonic acid or docosahexaenoic acid. It may be eicosanoids for example leukotrienes, prostanoids, prostaglandins, thromboxanes and/or prostacyclins. Preferably, the fatty acid is acetic acid.

In the present invention, the Z group can advantageously be an amino acid of the L or D series selected from the group comprising alanine, asparagine, an aromatic chain for example tyrosine, phenylalanine, tryptophan, thyroxine or histidine. Preferably, the amino acid is phenylalanine.

In the present invention, the Z group can be an antioxidant, for example vitamin A, C, E, B9, B6, glutathione, selenium, polyphenols, for example catechins, for example green tea, flavonoids, tannins, anthocyanins, for example fruit red, lycopene, terpenoids and resveratrol.

In the present invention, the Z group can be anti-aging compounds, for example retinoids, allantoins.

In the present invention, the Z group can be antibodies, antibody fragments, for example Fab fragments. It can be for example addressing antibodies and/or antibody fragments, for example antibodies and/or antibody fragments.

In the present invention, the Z group can be a fatty acid, for example such acetic acid.

Advantageously the Z groups can confer to the polymers additional biological or physicochemical properties. For example, the Z groups can increase the solubility or the lipophilicity of said polymer enabling for example a better diffusion or tissue penetration.

Advantageously, the Z groups can confer to the polymers additional biological or physicochemical properties. Thus, the polymers of the invention, for example when the Z group is selected from an antioxidant compound, the polymers of the invention can advantageously convey these compounds and thus provide an additional and/or complementary biological effect.

Polymers in which Z is present can have the following formula II: Aa Xx Yy Zz (II) wherein, A, X, Y a, x, y are as defined herein before and z is the rate of substitution by Z groups.

In the present invention the rates of substitution by Z groups represented by "z" can be from 1 to 50%, from 10 to 25%, preferably equal to 15, 20 or 25%.

The X, Y and Z groups can be independently bonded to the A monomer and/or independently bonded to one another. When at least one of the X, Y and Z groups is independently bonded to an X, Y and Z group other than the first, one of said X, Y or Z groups is bonded to the A monomer.

Thus, the Z groups can be bonded by covalence directly to the A monomers or bonded by covalence to the X and/or Y groups.

In the present invention the Z groups can also be conjugated with the polymers of formula AaXxYy by bonds other than covalent bonds, for example by ionic bonds, for example via ionic interactions, hydrophilic bonds or hydrophobic bonds. The polymers of the invention can then constitute a Z vectorization system.

In the present invention, the polymer can be for example a polymer selected from the group comprising the compounds OTR4120, OTR41201, OTR41202, OTR41203, OTR41205, OTR41210, OTR41301, OTR41302, OTR41303, OTR41305, OTR41310, OTR3131, OTR4131, OTR4132, preferably OTR4132.

In the present invention, the polymer can be for example a polymer selected from the group comprising the compounds OTR41201, OTR41202, OTR41203, OTR41205, OTR41210, OTR4120, OTR4122, OTR4125, OTR41301, OTR41302, OTR41303, OTR41305, OTR41310, OTR3131, OTR4131 OTR4132, OTR4135, OTR415 with the characteristics mentioned in table 1 below

### Table 1: list and characteristics of polymers

**Table 1: Polymers of the Aa Xx Yy (I) and Aa Xx Yy Zz (II) families wherein A is glucose (MW 180 Da), X is CarboxyMethyl (MW 58 Da) Y: SO₃⁻ (MW 80 Da) and Z is Acetate (MW 43 Da) or phenylalanine (MW 165 Da).**

| polymer | | A: glucose | X -CH₂COO⁻ | Y -SO₃⁻ | Z -OCCH₃ | Z phenylalanine |
|---|---|---|---|---|---|---|
| Name of the RGTA | Average molecular weight M or mass +/-15% (MW in Dalton) | Dextran starting polymer (MW in Dalton) | % substitution CM (X)/glucose | % substitution -SO₃⁻ (Y)/glucose) | % substitution OCCH₃ (Z)/glucose | % substitution phenylalanine (Z)/glucose |
| CMDS OTR41201 | 3000 | 1 500 | 60+/-20 | 150+/-20 | 0 | |
| CMDS OTR41202 | 6000 | 3000 | 60+/-20 | 150+/-20 | 0 | |
| CMDS OTR41203 | 10 000 | 5000 | 60+/-20 | 150+/-20 | 0 | |
| CMDS OTR41205 | 20 000 | 10 000 | 60+/-20 | 150+/-20 | 0 | |
| CMDS OTR41210 | 40 000 | 20 000 | 60+/-20 | 150+/-20 | 0 | |
| CMDS OTR4120 | 80 000 | 40 000 | 60+/-20 | 150+/-20 | 0 | |
| CMDS OTR4122 | 220 000 | 110 000 | 60+/-20 | 150+/-20 | 0 | |
| CMDS OTR4125 | 500 000 | 250 000 | 60+/-20 | 150+/-20 | 0 | |
| CMDSA OTR41301 | 3 000 | 1 500 | 60+/-20 | 140+/-20 | 20+/-5 | |
| CMDSA OTR41302 | 6000 | 3000 | 60+/-20 | 140+/-20 | 20+/-5 | |
| CMDSA OTR41303 | 10 000 | 5000 | 60+/-20 | 140+/-20 | 20+/-5 | |
| CMDSA OTR41305 | 20 000 | 10 000 | 60+/-20 | 140+/-20 | 20+/-5 | |
| CMDSA OTR41310 | 40 000 | 20 000 | 60+/-20 | 140+/-20 | 20+/-5 | |
| CMDSA OTR4131 | 80 000 | 40 000 | 60+/-20 | 140+/-20 | 20+/-5 | |
| CMDSA OTR4132 | 220 000 | 110 000 | 60+/-20 | 140+/-20 | 20+/-5 | |
| CMDSA OTR4135 | 500 000 | 250 000 | 60+/-20 | 140+/-20 | 20+/-5 | |
| CMDSP OTR415 | 5 000 | | 60+/-20 | 70+/-15 | - | 15+/-5 |

In the present "initial stroke lesion volume" means the volume of the stroke as measured within the first 48 hours following stroke onset, preferably within 24 hours following stroke onset.

In the present, the stroke lesion volume comprises the ischemic core of the stoke, the penumbra volume and/or the oedema volume, preferably, the stroke lesion volume comprises the ischemic core of the stoke, the penumbra volume and the oedema volume.

In the present, the determination of initial stroke lesion volume may be carried out by any method know from one skilled in the art. For example, it may be determined by the method disclosed in Ospel JM, Hill MD, Menon BK, Demchuk A, McTaggart R, Nogueira R, Poppe A, Haussen D, Qiu W, Mayank A, Almekhlafi M, Zerna C, Joshi M, Jayaraman M, Roy D, Rempel J, Buck B, Tymianski M, Goyal M; ESCAPE-NA1 investigators. Strength of Association between Infarct Volume and Clinical Outcome Depends on the Magnitude of Infarct Size: Results from the ESCAPE-NA1 Trial. AJNR Am J Neuroradiol. 2021 Aug;42(8):1375-1379. doi: 10.3174/ajnr.A7183. Epub 2021 Jun 24. PMID: 34167959; PMCID: PMC8367613 [16]. For example the determination of the stroke lesion volume may be assessed from MRI images using DICOM viewer software image segmentation and analysis tools comprising baseline measurements involved capturing ADC (Apparent Diffusion Coefficient) values using, for example, the Rapid software (iSchemaView, Menlo Park, CA), outlining the subacute FLAIR lesion with precision drawing tools, ensuring accurate demarcation of the lesion boundaries and following by calculating the total volume of these outlined regions which correspond to the total stroke lesion volume.

In the present, the determination of the initial stroke lesion volume can be measured within the first 48 hours following stroke onset, preferably within 24 hours following stroke onset.

In the present invention, the composition, for example for intra-arterial administration, can comprise a concentration of 0.01 to 3000 µg/mL by weight, for example of 0.1 to 300 µg/mL by weight of biocompatible polymer with respect to the volume of the composition. For example, the composition can comprise a concentration of 1 to 300 µg/mL, of 10 to 300 µg/ml, of 20 to 200 µg/mL by weight of biocompatible polymer with respect to the total volume of the composition.

In the present invention, the composition can be formulated so as to deliver, preferably intra-arterially, a final dose of biocompatible polymer of at least 1 mg in humans, whatever the initial concentration of the polymer, preferably 2 or 2.5 mg.

In the present invention, the composition can be formulated so as to deliver, preferably intra-arterially a final dose of biocompatible polymer of at least 10 µg.mL⁻¹ by weight of biocompatible polymer with respect to the total initial stroke lesion volume.

In the present invention, the composition can be formulated and/or adapted according to its administration.

For example, for intra-venous administration the composition can be administered so as to deliver a dose of biocompatible polymer comprised from 0.01 to 5 mg per kilogram of body weight (mg/kg), preferably from 0.1 to 1.5 mg per kilogram of body weight, for example with the frequency of one administration.

For example, for intra-venous administration, the composition can be formulated so as to deliver a dose of biocompatible polymer of at least 100 µg.mL⁻¹, preferably 200 µg.mL⁻¹ by weight of biocompatible polymer with respect to the total initial stroke lesion volume.

For example, for oral administration the composition can be administered so as to deliver a dose of biocompatible polymer comprised from 0.03 to 15 mg per kilogram of body weight (mg/kg), preferably from 0.3 to 4.5 mg/kg per kilogram of body weight, for example with the frequency of one daily or biweekly administration.

In the present, "pharmaceutical composition" means any form of pharmaceutical composition known to a skilled person. In the present document, the pharmaceutical composition can be for example an injectable solution. It can be for example an injectable solution, for example for a local or systemic injection, for example in physiological serum, in injectable glucose solution, in the presence of excipients, for example of dextrans, for example at concentrations known to the skilled person, for example from one microgram to several milligrams per mL. The pharmaceutical composition can be for example a drug intended for oral administration selected from the group comprising a liquid formulation, an oral effervescent dosage form, an oral powder, a multiparticle system, an orodispersible dosage form.

The composition of the present invention may also comprise at least one other active ingredient, particularly another therapeutically active ingredient or therapeutic agents, for example for simultaneous or separate use or for use spread out over time depending on the galenic formulation used. This other ingredient can be for example an active ingredient or therapeutic agent used for example in the treatment of ischemic stroke. It can be for example a therapeutic agent known to the skilled person and/or commercially available, for example selected from the group comprising fibrinolytics, anticoagulant, anti-aggregants or other neuroprotective drugs.

The fibrinolytic can be for example any fibrinolytics adapted and commercially available known from one skilled in the art. It may be for example a fibrinolytics selected from the group comprising streptokinase, urokinase alteplase, Tenecteplase, reteplase, tranexamic acid, aminocaproic acid, nafamostat and aprotinin.

The anticoagulant can be for example any anticoagulant adapted and commercially available known from one skilled in the art. It may be for example a anticoagulant selected from the group comprising apixaban, dabigatran, edoxaban, rivaroxaban, warfarin, heparin, dalteparine, enoxaparine, tinzaparine, fondaparinux.

The anti-aggregant can be for example any anti-aggregant adapted and commercially available known from one skilled in the art. It may be for example a , anti-aggregant selected from the group comprising acetylsalicylic acid, clopidogrel, prasugrel, dipyridamole, ticagrelor, cangrelor.

The neuroprotective drug can be for example any neuroprotective drug adapted and commercially available known from one skilled in the art.

The composition of the present invention can also comprise at least one other active ingredient, particularly another therapeutically active ingredient, for example for simultaneous, separate or time-staggered use depending on the galenic formulation used. This other ingredient may be, for example, stem cells with the exception of embryonic stem cells.

According to the invention, the composition can be, for example, administered only once.

According to the invention, the composition can further be, for example, administered daily, bi-daily and weekly or less. It can be for example administration once a day, twice a day or less, for example once every other day, or weekly.

According to the invention, and the mode of administration the composition can be, for example, for a saline composition administered daily, bi-daily and weekly or less. It can be for example administered once daily, twice daily or less.

In the present, the composition of the present invention can be used in a combination therapy, for example with other active ingredient, particularly another therapeutically active ingredient or therapeutic agent.

"Combination therapy" (or "co-therapy") includes the administration of a pharmaceutical composition of the invention, and at least a second agent, for example an active ingredient, particularly another therapeutically active ingredient or therapeutic agent, as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically can be carried out over a defined time period (usually minutes, hours, days or weeks depending upon the combination selected).

"Combination therapy" can, but generally is not, intended to encompass the administration of two or more of these therapeutic agents as part of separate monotherapy regimens that incidentally and arbitrarily result in the combinations of the present invention. "Combination therapy" can embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a pharmaceutical composition having a fixed ratio of each therapeutic agent or in multiple, pharmaceutical compositions for each of the therapeutic agents. Sequential or substantially simultaneous administration of each therapeutic agent can be effected by any appropriate route including, but not limited to, topical routes, oral routes, intravenous routes, intraarterial routes and direct absorption through mucous membrane tissues. The therapeutic agents can be administered by the same route or by different routes. For example, a first therapeutic agent, for example the biocompatible polymer of the invention, of the combination selected can be administered by injection, for example via intravenous route and/or intraarterial route, while the other therapeutic agents of the combination may be administered orally.

The therapeutic agent can be for example selected from the group comprising fibrinolytics, anticoagulant, anti-aggregants, neuroprotective drugs and any combinations thereof.

The fibrinolytics, anticoagulant, anti-aggregants or other neuroprotective drugs can be as mentioned above.

The present invention also relates to a method of treating a patient who has ischemic stroke, comprising administration of a pharmaceutical composition comprising a biocompatible polymer of formula AaXxYy or AaXxYyZz.

According to the invention, the biocompatible polymer of formula AaXxYy or AaXxYyZz is as defined above.

According to the invention, the pharmaceutical composition is as defined above.

According to the invention, the dose to be administered may be as defined above.

According to the invention, the frequency of administration of the pharmaceutical composition may be as defined above.

According to the invention, the mode and/or route of administration of the pharmaceutical composition may be as defined above.

According to the invention, the patient or subject may be any mammal. It may, for example, be an animal or a human being.

According to the invention, the method of treatment can comprise, before the administration of the pharmaceutical composition comprising a biocompatible polymer of formula AaXxYy or AaXxYyZz with a step of determination of the initial stroke lesion volume.

The determination of the initial stroke lesion volume may be carried out within 48 hours, preferably within 24 hours following stroke onset by any method know from one skilled in the art. For example, it may be determined by the method disclosed in Ospel JM, Hill MD, Menon BK, Demchuk A, McTaggart R, Nogueira R, Poppe A, Haussen D, Qiu W, Mayank A, Almekhlafi M, Zerna C, Joshi M, Jayaraman M, Roy D, Rempel J, Buck B, Tymianski M, Goyal M; ESCAPE-NA1 investigators. Strength of Association between Infarct Volume and Clinical Outcome Depends on the Magnitude of Infarct Size: Results from the ESCAPE-NA1 Trial. AJNR Am J Neuroradiol. 2021 Aug;42(8):1375-1379. doi: 10.3174/ajnr.A7183. Epub 2021 Jun 24. PMID: 34167959; PMCID: PMC8367613 [16]. For example the determination of the infarct volume may be assessed from MRI images using DICOM viewer software image segmentation and analysis tools comprising baseline measurements involved capturing ADC (Apparent Diffusion Coefficient) values using, for example, the Rapid software (iSchemaView, Menlo Park, CA), outlining the subacute FLAIR lesion with precision drawing tools, ensuring accurate demarcation of the lesion boundaries and following by calculating the total volume of these outlined regions which correspond to the total infarct volume.

According to the invention, pharmaceutical composition can be formulated so as to administer, for example intra-arterially a concentration of biocompatible polymer of at least 10 µg.mL⁻¹ by weight of biocompatible polymer with respect to the initial stroke lesion volume. For example, the concentration of the biopolymer of formula AaXxYy or AaXxYyZz can be from 10 µg.mL⁻¹ to 1000 µg.mL⁻¹ by weight of biocompatible polymer with respect to the initial volume of the stoke lesion.

According to the invention, pharmaceutical composition can be formulated so as to administer, for example intra-venously, a concentration of biocompatible polymer of at least 200 µg.mL⁻¹ by weight of biocompatible polymer with respect to the initial stroke lesion volume. For example, the concentration of the biopolymer of formula AaXxYy or AaXxYyZz can be from 200 µg.mL⁻¹ to 1000 µg.mL⁻¹ by weight of biocompatible polymer with respect to the total initial volume of the stoke.

According to the invention, the method of treatment can further comprise, a further administration step (i) of at least one further active ingredient, particularly another therapeutically active ingredient or therapeutic agent. The at least one further active ingredient can be a therapeutic agent for example selected from the group comprising fibrinolytics, anticoagulant, anti-aggregants, neuroprotective drugs and any combinations thereof. The fibrinolytics, anticoagulant, anti-aggregants or other neuroprotective drugs can be as mentioned above. The further administration step (i) can be carried out before, simultaneously or after, the administration of a pharmaceutical composition comprising a biocompatible polymer of formula AaXxYy or AaXxYyZz.

### Equivalents

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

### Exemplification

The present invention and its applications can be understood further by the examples that illustrate some of the embodiments by which the inventive medical use may be reduced to practice. It will be appreciated, however, that these examples do not limit the invention. Variations of the invention, now known or further developed, are considered to fall within the scope of the present invention as described herein and as hereinafter claimed. Other advantages may further emerge to those skilled in the art on reading the examples below, illustrated by the appended figures, and given by way of illustration.

### Brief description of the figures

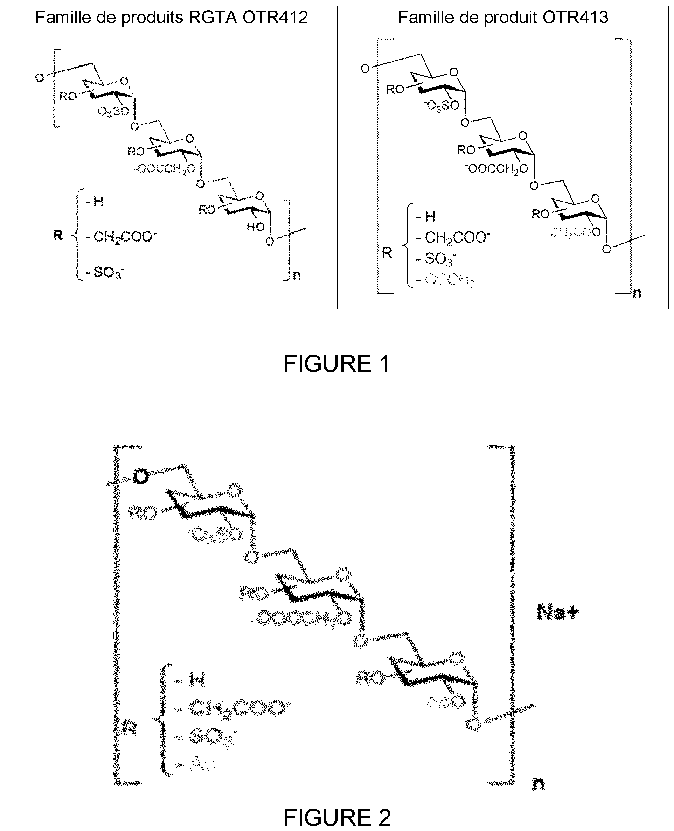

Figure 1 shows examples of the structure of the biocompatible polymer, for example the structure of the families of compounds OTR412 and OTR413, and OTR415
Figure 2 shows examples of the structure of the biocompatible polymer OTR4132.

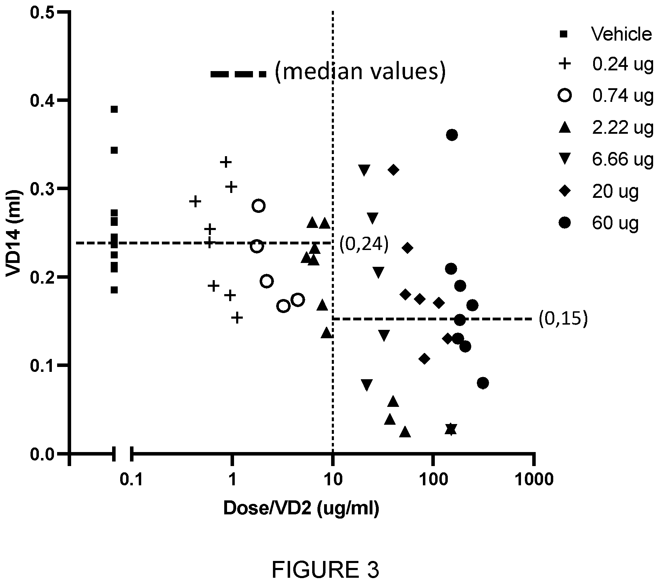

Figure 3 is a diagram representing stroke lesion volume at Day 14 (VD14) in ml (ordinate) in rats as a function of initial dose administered intra-arterially per lesion volume at Day 2 in µg/ml (abscissa) as described in example 1. In the figure, ■ (square) means no OTR4132, + (plus sign) means total dose of 0,24 µg of OTR4132, ∘ (circle) means total dose of 0.74µg of OTR4132, A (triangle up) means total dose of 2.22µg of OTR4132, ▼ (triangle down) means total dose of 6.66µg of OTR4132, ◆ (lozenge) means total dose of 20µg of OTR4132 and • (round) means total dose of 60µg of OTR4132. In the figure doses/volume are represented in a logarithmic scale

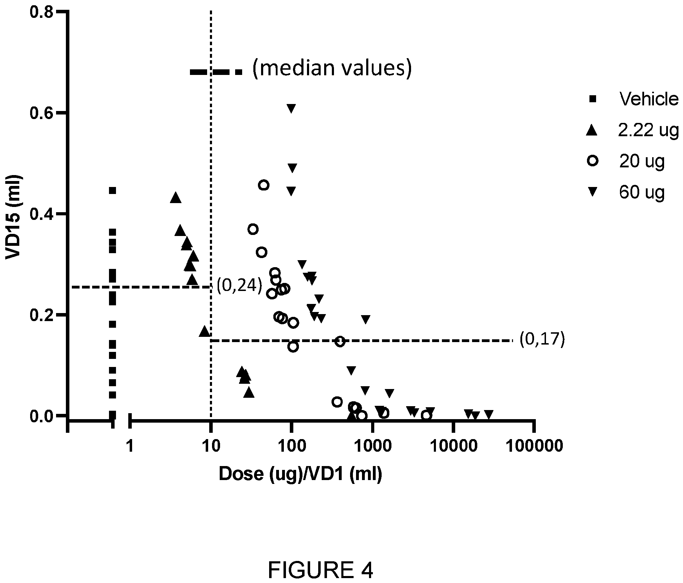

Figure 4 is a diagram representing stroke lesion volume at Day 15 (VD15) in ml (ordinate) in rats as a function of initial dose administered intra-arterially per lesion volume at Day 1 in µg/ml (abscissa) as described in example 2. In the figure, ▲ (triangle up) means total dose of 2.22µg of OTR4132, • (round) means total dose of 60µg of OTR4132, ∘ means total dose of 20µg of OTR4132 ▼ (triangle down) means total dose of 60 µg of OTR4132 and ■ (square) means no OTR4132. In the figure doses/volume are represented in a logarithmic scale.

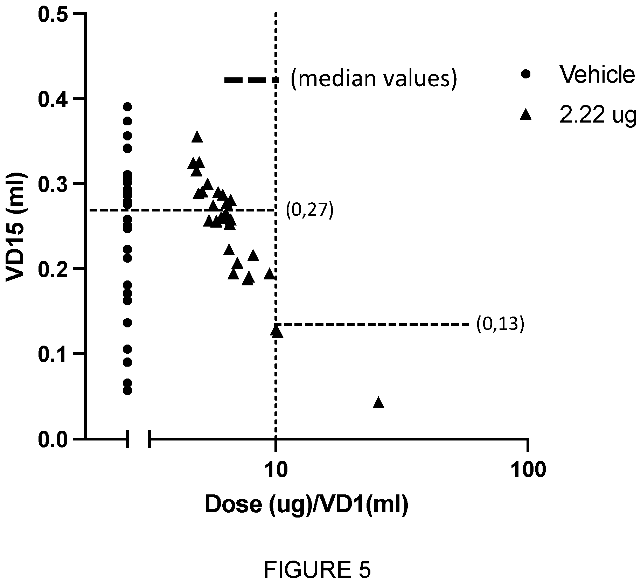

Figure 5 is a diagram representing stroke lesion volume at Day 15 in ml (ordinate) related to D1 in rats as a function of initial dose administered intra-arterially per total lesion volume at Day 1 in µg/ml (abscissa) ) as described in example 3. In the figure ▲ (triangle up) means total dose of 2.22µg of OTR4132 and ■ (square) means no OTR4132. In the figure doses/volume are represented in a logarithmic scale.

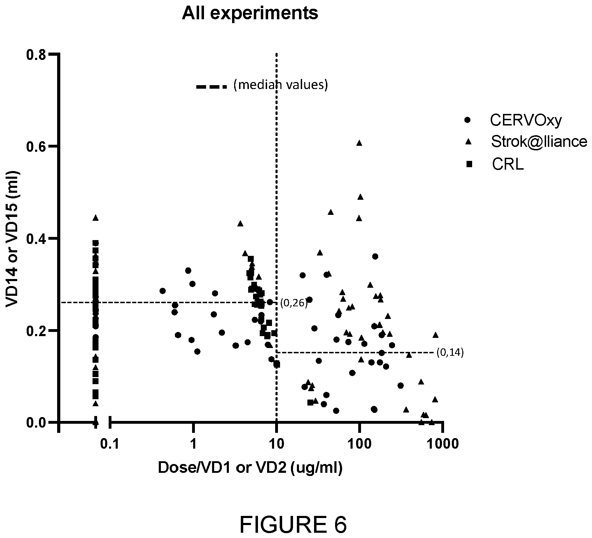

Figure 6 is a meta-analysis from the 3 experiments in rats (examples 1, 2 and 3 corresponding to the results disclosed in figures 3 to 5). The diagram representing the ratio between total infarct volume at Day 14 (or 15) in ml (ordinate) in rats related to D1 (or D2) as a function of initial dose administered intra-arterially per total lesion volume at Day 1 (or D2) in µg/ml (abscissa).
In the figure, • (round) in first study ▲ (triangle up) in second study and ■ (square) in third study. In the figure doses/volume are represented in a logarithmic scale.

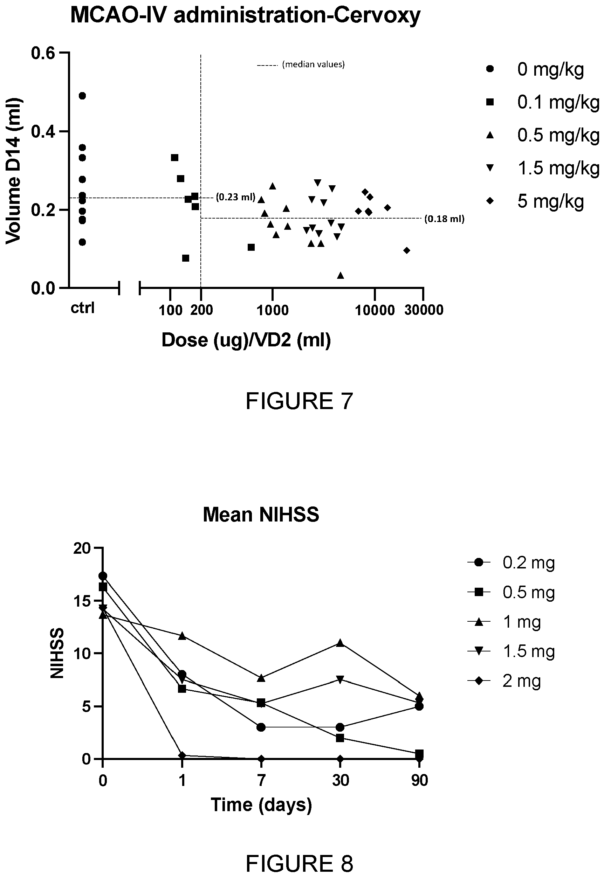

Figure 7 is a diagram representing stroke lesion volume at Day 14 (VD14) in ml (ordinate) in rats as a function of initial dose of OTR4131 (HSm4131) administered intra-venously per lesion volume at Day 2 in µg/ml (abscissa) as described in example 4. In the figure, ■ (square) means total dose of 0.1 mg/kg of HSm4131, ▲ (triangle up) means total dose of 0.5 mg/kg of HSm4131, ▼ (triangle down) means total dose of 1.5 mg/kg of HSm4131, ◆ (lozenge) means total dose of 5 mg/kg of HSm4131 and • (round) means no HSm4131. In the figure doses/volume are represented in a logarithmic scale.
Figure 8 is a diagram representing curves of results of NIH Stroke Scale (NIHSS) in humans according to time. Abscissa represents time, with Visit 1: 24 hours, Visit 2: 7 days, Visit: 1 month and Visit: 3 months per patient. Ordinate NIH Stroke Scale (NIHSS). Measures were taken at each visit time for five dose group, i.e. a dose of 0.2 mg of OTR4132 (round), a dose of 0.5 mg of OTR4132 (square), a dose of 1 mg of OTR4132 (triangle up), a dose of 1.5 mg of OTR4132 (triangle down) or a dose of 2 mg of OTR4132 (lozenge). The NIH Stroke Scale (NIHSS) is based on the collection of 15 clinical neurological items. It allows for an accurate and rapid assessment of observed deficits. A large number of publications have shown that the NIHSS score at 24 hours is the best prognostic factor for long-term functional disability and is closely correlated with disability scores at 3 months. An NIHSS score between 1 and 4 means a minor stroke, between 5 and 15, a moderate stroke, between 15 and 20, severe, and above 20 points, a severe stroke. The maximum score is 42.

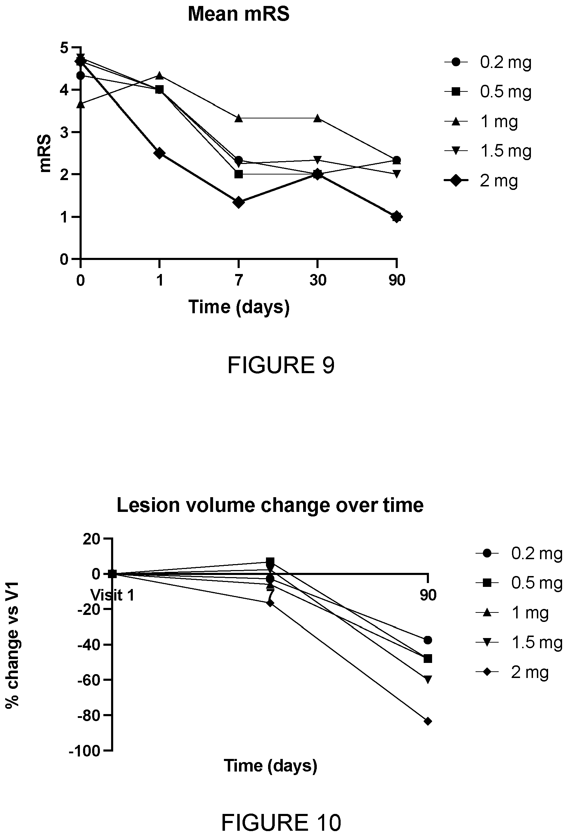

Figure 9 is a diagram representing curves of results of modified RANKIN score (mRS) in humans according to time. Abscissa represents time with Visit 1: 24 hours (1 day), Visit 2: 7 days, Visit: 1 month (30 days) and Visit: 3 months (90 days). Ordinate correspond to value of modified RANKIN score (mRS). Measures were taken at each visit time for five patient and dose group, i.e. a dose of 0.2 mg of OTR4132 (round), a dose of 0.5 mg of OTR4132 (square), a dose of 1 mg of OTR4132 (triangle up), a dose of 1.5 mg of OTR4132 (triangle down) or a dose of 2 mg of OTR4132 (lozenge). On this diagram, value of modified RANKIN score (mRS) equal to "0" means No symptoms, "1" means no disability apart from symptoms: activities and autonomy maintained, "2" means low disability: unable to perform usual activities but independent, "3" means moderate disability: needs help but walks without assistance, "4" means moderately severe disability: walking and daily movements impossible without assistance and "5" means major disability: permanent bed rest, incontinence and permanent nursing care.
Figure 10 is a diagram representing the change in the stroke lesion volume in humans according to time. Ordinate represents proportion of changes at 3 months compared to initial stroke lesion volume measured at V1 (24 hours). Abscissa shows the dose of OTR4132: 0.2 mg of OTR4132 (round), a dose of 0.5 mg of OTR4132 (square), a dose of 1 mg of OTR4132 (triangle up), a dose of 1.5 mg of OTR4132 (triangle down) or a dose of 2 mg of OTR4132 (lozenge). %Median change values are shown.

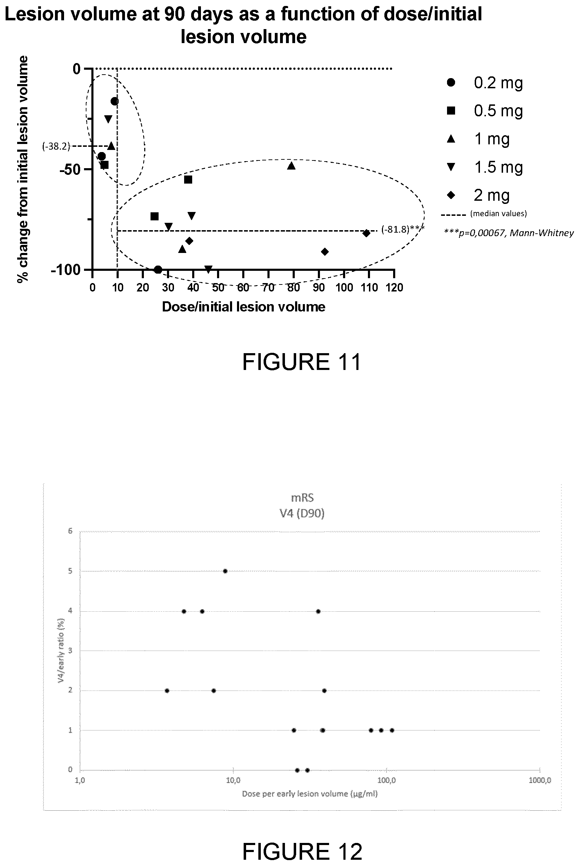

Figure 11 is a diagram representing the % stroke lesion volume ("lesion volume") change in humans at 3 months compared to initial stroke lesion volume (ordinate) as a function of the ratio of total dose administered/initial lesion (abscissa) in µg/ml. Dose group are : 0,2 mg of OTR4132 (round), 0,5 mg of OTR4132 (square), 1 mg of OTR4132 (triangle up), 1,5 mg of OTR4132 (triangle down) and 2 mg of OTR4132 (lozenge).
Figure 12 is a diagram representing the mRS scores at 3 months in humans compared to early values (ordinate) as a function of the ratio of total dose administered/ initial stroke lesion volume (abscissa) in µg/ml.

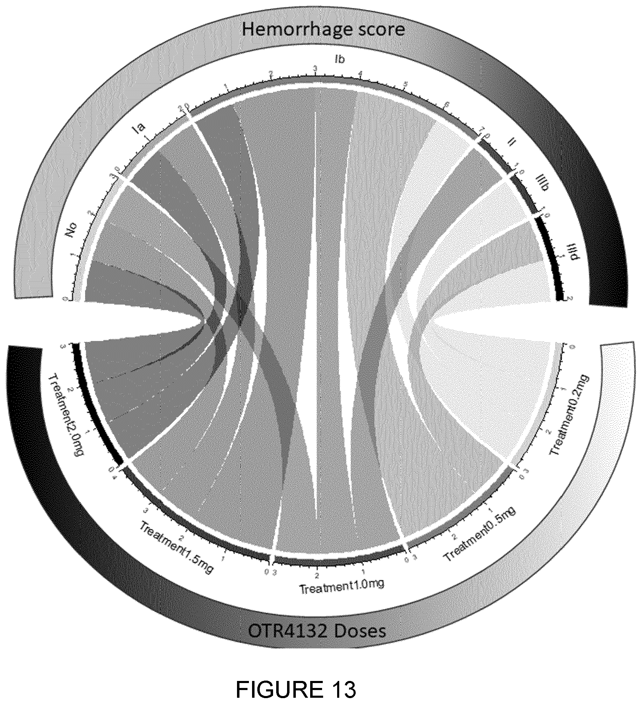

Figure 13 is a CIRCOS graph representing the rate of hemorrhagic transformation at 24 hours in humans using the Heidelberg classification according to the dose of OTR4132: 0.2 mg of OTR4132, a dose of 0.5 mg of OTR4132, a dose of 1 mg of OTR4132, a dose of 1.5 mg of OTR4132 or a dose of 2 mg of OTR4132. Heidelberg Bleeding Classification: NO: absence of intracranial hemorrhage; Ia: scattered small petechiae, no mass effect; Ib: confluent petechiae, no mass effect; II: intracerebral hemorrhage within and beyond infarcted brain tissue; IIIb: intraventricular hemorrhage; IIId: subdural hemorrhage. Only categories found in the study cohort are shown. The graph shows that higher doses correlate with the less severe hemorrhages.

### Examples

### Example: Effect of biocompatible polymer in the treatment of ischemic stroke, in rats

### A/ Preparation of biocompatible polymers

The synthesis of biocompatible polymers, RGTA, is widely described in the prior art, for example in patent US7396923 [22] entitled "process for sulfonation of compounds comprising free hydroxyl (OH) groups or primary or secondary amines" and also in the bibliographic reference Yasunori I. et al., Biomaterials 2011, 32 :769e776 [20]) and Petit E. et al,. Biomacromolecules. 2004 Mar-Apr; 5(2):445-52 [21].

The biocompatible polymer used in the experiment is OTR4132, also designated OTR4132-MD herein, is represented in figure 2 and mentioned in table 2 below :

| | | | | | |
|---|---|---|---|---|---|
| polymer | | A: glucose | X -CH₂COO⁻ | Y -SO₃⁻ | Z -OCCH₃ |

| Name of the RGTA | Average molecular weight M or mass +/-15% (MW in Dalton) | Dextran starting polymer (MW in Dalton) | % substitution CM (X)/glucose | % substitution -SO₃⁻ (Y)/glucose) | % substitution OCCH₃ (Z)/glucose |
|---|---|---|---|---|---|
| CMDSA OTR4132 | 220 000 | 110 000 | 60+/-20 | 140+/-20 | 20+/-5 |

The compound OTR4132, also designated OTR4132-MD, herein is a compound comprising a Z radical which is a fatty acid, namely acetic acid

### B/ Effect of biocompatible polymer in rats following stroke

OTR4132-MD has been investigated in preclinical studies to assess its neuroprotective potential in Acute Ischemic Stroke in rat.

In these examples, procedures were performed in compliance with 3R's ethical rules on animal experimentation and in accordance with the European Directive of 22 September 2010 concerning animal experimentation (2010/63/EU). The protocol was submitted to ethic approval of the Ministry of Higher Education and Research to obtain a project authorization for using animals for scientific purposes.

### B/1. First study

In this example, the experimental procedures were performed at the CYCERON platform (Caen, France, agreement number: FA-118-001) on 300-350g weighing male Sprague Dawley (SD) rats (CERJ, France).

After the induction of a transient cerebral ischemia, animals underwent an MRI examination to measure the stroke lesion volume lesion at both day 2 and day 14 after ischemic stroke. All experiments and data analysis were performed in a blind and randomized manner according to ARRIVE (Animal Research: Reporting of In Vivo Experiments) guidelines. The randomization of the animals was performed using "Alea" function on Excel which permits a random allocation of the animals through the groups studied. The quantification of the lesions on MRI was performed by an experimenter who was blind to the treatment.

Transient cerebral ischemia was induced by intraluminal occlusion of the middle cerebral artery (MCAo) as described in Chuquet et al., 2002 [4]; Quittet et al., 2015 [15]. Briefly, rats were anesthetized with isoflurane (2-2.5%) in a mixture of O₂/N₂O (30%/70%). During surgery, body temperature was monitored with a rectal probe and was maintained at 37°C with a heating blanket. A nylon filament (0.18 mm diameter) with a distal cylinder (0.39 mm diameter × 3 mm length) (Doccol, Sharon, MA, USA) was introduced into the lumen of the right external carotid, advanced through the internal carotid, and gently pushed up to the origin of the middle cerebral artery (MCA). At one hour following the occlusion, rats were re-anesthetized and the filament was withdrawn to allow reperfusion. After reperfusion, the rats were returned to their home cage after receiving analgesics (buprenorphine, buprecare 0.3 mg/ml, s.c.) and saline (5 mL of saline, i.p.). Analgesics and saline (to avoid any dehydratation) were repeated daily during the 3 days after MCAo.

OTR4132-MD or saline (0.9% NaCl) were injected (50 µL) through the internal carotid artery during 2 min, through a catheter connected to a 50µL Hamilton syringe. To evaluate the dose response, different doses of OTR4132, i.e., 0.24 µg, 0.74 µg, 2.22 µg, 6.66 µg, 20 µg or 60 µg were similarly administered 1 h after MCAo. The used rats were Sprague-Dawley, the administration of OTR4132 was carried out on a population of 57 rats including 12 vehicle-treated rats.

The respective concentrations are 4.8, 14.8, 44.4, 133.2, 400 and 1200 µg/ml. Each solution was prepared the day of the experiment. 700 µg of OTR4132 was suspended in 1 ml saline and then diluted in saline to reach the desired concentration.

The effects of OTR4132-MD on stroke-induced brain damage were quantified using MRI both at 2 and 14 days following the insult: on day 2 and 14 following the induction of brain ischemia, each animal was anesthetized as described above and underwent magnetic resonance imaging (MRI) (7T, PharmaScan^{®}, Bruker BioSpin, Ettlingen, Germany at the CYCERON imaging platform, Caen, France). After a scout view, T2w imaging was performed for each group with a rapid acquisition with refocused echoes (RARE) sequence (RARE factor of 8; TRITE = 5000/16.25 ms; number of experiments (NEX) = 2; 20 contiguous slices of 0.75 mm; acquisition time = 4 min; nominal resolution = 0.15 × 0.15 × 0.75 mm³). All MRI images were analyzed with the ImageJ^{®} software (Wayne Rasband, NIMH, Maryland, USA).

Post-hoc analyses were performed to study the stroke lesion volume at day 14 according to the dose/initial lesion volume. Raw data for lesion volumes were reported in % of brain hemisphere. To estimate the exact lesion volume, we assumed from the literature that a brain hemisphere in Sprague Dawley rats is 0.88 ml. In this set of experiments, brain MRIs were performed at Day 2 and Day 14. Day 2 volumes were used to estimate the total dose administered per initial lesion volume. Results of brain volumes at Day 14 are shown in figure 3:
Stroke lesion volumes in animals that received less than 10 µg/ml (median lesion volume value of 0.236 ml, n=32) were significantly higher than stroke lesion volumes in animals that received more than 10 µg/ml (median lesion volume value of 0.151 ml, n=25), p=0.00028. Of note, the dose of 2.2 mg which was supposed to be the most efficacious in these experiments is in fact at the limit of the 10 µg/ml threshold.

### B/ 2. Second study

A total of 140 adult male Sprague-Dawley rats (200-220 g, 5-6 weeks of age) were supplied by Janvier Lab (Le Genest-Saint-Isle, France). All rats were handled in the same way and under the same conditions.

The 70 rats after confirmed for eligibility on day 0, were allocated to 5 groups of treatments (n = 14/group, with n meaning number of animals used). A total of 70 additional rats were used to complete experimental groups in case of technical problem or exclusion criterion.

The randomization of the animals was performed by unrestricted random sampling according to a discrete uniform distribution with Excel 2003 for Windows (Microsoft, Bethesda, USA) using the function RANDBETWEEN.

In this example, focal ischemia was induced in adult male Sprague-Dawley rats by insertion of a nylon filament (0.18 mm diameter) with a distal cylinder (0.39 mm diameter × 3 mm length) (Doccol, Sharon, MA, USA) through the internal carotid artery (ICA) up to right middle cerebral artery (MCA) birth at Willis' circle level. Ischemia duration is accurately controlled since reperfusion is allowed by filament removal after 1h of MCA occlusion (MCAo). Rats were anesthetized with isoflurane (4,5% for induction, 2% for maintenance) in a mixture of O₂/N₂O (30%/70% by volume). During surgery, rectal temperature was maintained at 37°C with a feed-back controlled heating pad.

OTR4132-MD (50 µL of OTR4132 diluted in saline solution 0.9%) was intra-arterially (I.A.) administered 10 minutes (min) after the second perfusion in ischemic rats, through a catheter connected to a 50µL Hamilton syringe. The treatment was tested at 3 doses (2.22, 20 and 60 µg per animal) and compared to a vehicle-treated group (saline solution 0.9%). A sham group was used to compare the effects of MCAo from the effect of surgery and treatment procedures.

After the second perfusion, the rats were returned to their home cage after receiving analgesics (buprenorphine, buprecare 0.05 mg/ml, s.c.).

To prepare injectable solution, a stock solution was prepared from lyophilized OTR4132 (OTR3) at a concentration of 1.2 mg/mL. The solution was filtered two times with 0.2 µm filters (Minisart Plus 17823K, Sartorius) connected to a sterile syringe (Terumo). The stock solution was then diluted in saline solution to reach the desired concentration. The three prepared solutions were stored at +4°C in sterile screw-caped cryotubes and warmed at room temperature before injection.

Effects of OTR4132-MD on stroke lesion volume were quantified through the use of magnetic resonance imaging (MRI) at 1 and 15 days following the insult: on day 1 and 15 following the induction of brain ischemia, each animal was anesthetized as described above and underwent MRI (7T, PharmaScan^{®}, Bruker BioSpin, Ettlingen, Germany at the CYCERON imaging platform, Caen, France) with volume transmit and surface receive coils and piloted by Paravision^{©} v6.

After a scout view, T2-weighted (T2w) imaging was performed for each group with a rapid acquisition with refocused echoes (RARE) sequence (parameters: Relaxation time (TR)/ Echo Time (TE) 50/2500 ms, Echo Spacing: 12.5 ms, Rare factor 8°, Field of View = 25 × 35 × 20 mm, Matrix = 250 × 350 ; 20 contiguous slices of 1 mm; nominal resolution = 0.1 × 0.1 × 1 mm³). All MRI images were analysed with the ImageJ^{®} software (Wayne Rasband, NIMH, Maryland, USA).

Post-hoc analyses were performed to study the stroke lesion volume at day 15 according to the dose/initial stroke lesion volume. Results of total stroke lesion volumes at Day 15 are shown in figure 4. Day 1 volumes were used to estimate the total dose administered per stroke lesion volume. Although there was a huge variability in vehicle-treated animals in this set of experiments, total stroke lesion volumes in animals that received less than 10 µg/ml (median lesion volume value of 0.24 ml, n=29) were nevertheless significantly higher than stroke lesion volumes in animals that received more than 10 µg/ml (median lesion volume value of 0.081 ml, n=51), p=0.0125, Mann-Whitney.

### B/ 3. Third study

OTR4132-MD has been investigated in a third study to assess its neuroprotective potential in Acute Ischemic Stroke in rat.

After the induction of a transient cerebral ischemia, animals underwent an MRI examination to measure the stroke lesion volume lesion at both day 1 and day 15 after ischemic stroke. All experiments and data analysis were performed in a blind and randomized manner according to ARRIVE (Animal Research: Reporting of In Vivo Experiments) guidelines. The randomization of the animals was performed using "Alea" function on Excel which permits a random allocation of the animals through the groups studied. The quantification of the lesions on MRI was performed by an experimenter who was blind to the treatment as disclosed in above examples 1 and 2.

Transient cerebral ischemia was induced by intraluminal occlusion of the middle cerebral artery (MCAo) as described above in Chuquet et al., 2002 [4]; Quittet et al., 2015 [15]. Briefly, rats were anesthetized with isoflurane (2-2.5%) in a mixture of O₂/N₂O (30%/70%). During surgery, body temperature was monitored with a rectal probe and was maintained at 37°C with a heating blanket. A nylon filament (0.18 mm diameter) with a distal cylinder (0.39 mm diameter × 3 mm length) (Doccol, Sharon, MA, USA) was introduced into the lumen of the right external carotid, advanced through the internal carotid, and gently pushed up to the origin of the middle cerebral artery (MCA). At one hour and a half following the occlusion, rats were re-anesthetized and the filament was withdrawn to allow reperfusion. After reperfusion, the rats were returned to their home cage after receiving analgesics (buprenorphine, buprecare 0.3 mg/ml, s.c.) and saline (5 mL of saline, i.p.). Analgesics and saline (to avoid any dehydratation) were repeated daily during the 3 days after MCAo.

OTR4132-MD or saline (0.9% NaCl) were injected (50 µL) through the internal carotid artery during 2 min, through a catheter connected to a 50µL Hamilton syringe. A dose of OTR4132 at 2.22 µg was similarly administered intra-arterially in 29 rats and compared to 28 vehicles. The used rats were Wistar rats.

Post-hoc analyses were performed to study the stroke lesion volume at day 15 according to the dose/initial stroke lesion volume. The obtained results is disclosed in figure 15 showing the total stroke lesion volume at Day 15 (in ml) as a function of initial dose administered per total stroke lesion volume at Day 1 (in µg/ml).

This set of experiments did not show any statistical superiority of OTR4132-MD over vehicle.

However, as shown in figure 5, the dose/stroke lesion volume of 10 µg/ml was never reached in this set of experiments except in 3 animals that showed the lowest stroke lesion volume at Day 15. The median stroke lesion volume at Day 15 in animals exposed to less than 10 µg/ml was 0.27 whereas the median volume at Day 15 in the 3 animals exposed to 10 µg/ml or more was 0.13.

A post-hoc analysis of all the results obtained in examples 1 - 3 (figures 3-5) regarding the total stroke lesion volume at Day 14 or Day 15 (in ml) as a function of initial dose administered per total stroke lesion volume at Day 1 or Day2 (in µg/ml) has been carried out.

In other words, a meta-analysis of all available data (n=194) obtained from example 1 - 3 above has been carried out. The results obtained are represented in figure 6 showing the effects on stroke lesion volume at the last available evaluation (Day 14 or Day 15) as a function of the initial dose administered per total stroke lesion volume (D1 or D2). As demonstrated in this figure, stroke lesion volumes in animals that received less than 10 µg/ml of stroke lesion volumes (median lesion volume value of 0.26 ml) were significantly higher than stroke lesion volumes in animals that received more than 10 µg/ml stroke lesion volumes (median lesion volume of 0.14 ml), p=4 e-7.

The results clearly confirm that the use of OTR4132 for the treatment of stroke allows surprisingly and unexpectedly, to reduce the volume of the stroke lesion.

As demonstrated above, for example in figure 6, the in vivo experiments clearly demonstrate that OTR4132 allow the treatment of ischemic stroke. In particular, the results clearly demonstrate that OTR4132 administered intra-arterially provides a surprising therapeutical effect above the dose of 10 µg/ml of stroke lesion volume.

### B/ 4. Fourth study

OTR4131 also designated HSm4131 has been investigated in a study to assess its neuroprotective potential in Acute Ischemic Stroke in rat following intravenous administration. In this study, data were re-analysed in post hoc analyses to study the effects of OTR4131 as a function of dose administered per stroke lesion volume.

Transient cerebral ischemia was induced by intraluminal occlusion of the middle cerebral artery (MCAo) as previously described. Briefly, rats were anesthetized with isoflurane (2-2.5%) in a mixture of O₂/N₂O (30%/70%). During surgery, animal temperature was monitored with a rectal probe and was maintained at 37 °C with a heating blanket. A nylon filament (0.18 mm diameter) with a distal cylinder (0.39 mm diameter × 3 mm length) (Doccol, Sharon, MA, USA) was introduced into the lumen of the right external carotid, advanced through the internal carotid, and gently pushed up to the origin of the middle cerebral artery (MCA). At one hour following the occlusion, rats were re-anesthetized and the filament was withdrawn to allow reperfusion. After reperfusion, the rats were returned to their home cage after receiving analgesics (tolfedine, 10 mg/kg, i.m.) and rehydration (5 mL of saline, i.p.), which was maintained for 3 days after MCAo.

As mentioned above, OTR4131 also designated HSm4131 (OTR3 S.A.S., Paris, France) is a 132 kDa synthetic polysaccharide. HSm4131 or saline (0.9% NaCl) were injected (300 µL) through the tail vein. To evaluate the dose response, different doses of HSm4131, i.e., 0.1 mg/kg (0.7 nmol/kg), 0.5 mg/kg (3.7 nmol/kg), 1.5 mg/kg (11.3 nmol/kg), or 5 mg/kg (37.9 nmol/kg) were similarly administered 1 h after MCAo.

On day 2 and 14 following the induction of brain ischemia, each animal was anesthetized as described above and underwent magnetic resonance imaging (MRI) (7T, PharmaScan^{®}, Bruker BioSpin, Ettlingen, Germany at the CYCERON imaging platform, Caen, France). After a scout view, T2w imaging was performed for each group with a rapid acquisition with refocused echoes (RARE) sequence (RARE factor of 8; TRITE = 5000/16.25 ms; number of experiments (NEX) = 2; 20 contiguous slices of 0.75 mm; acquisition time = 4 min; nominal resolution = 0.15 × 0.15 × 0.75 mm³). All MRI images were analysed with the ImageJ^{®} software (Wayne Rasband, NIMH, Maryland, USA). Animals displaying atypical lesions for the MCAo model (i.e., no cortical lesion, lesion in the hippocampus) were excluded from the study.

The stroke lesion volume at day 14 according to the dose/initial stroke lesion volume has been studied. The obtained results are disclosed in figure 7 showing the total stroke lesion volume at Day 14 (in ml) as a function of initial dose administered per total stroke lesion volume at Day 2 (in µg/ml).

Total stroke lesion volumes in animals that received less than 200 µg/ml (median lesion volume value of 0.23 ml, n=17) were significantly higher than stroke lesion volumes in animals that received more than 200 µg/ml (median lesion volume value of 1.8 ml, n=28), p=0.004, Mann-Whitney.

These in vivo experiments demonstrate that OTR4131 allows the treatment of stroke, in particular the reduction of the stroke lesion volume. In addition, the results showed in figure 7 clearly demonstrate that OTR4131 administered intra-venously provides a surprising therapeutical effect at the dose/concentration above 200 µg/ml of initial stroke lesion volume.

### Example 2: Effect of biocompatible polymer in the treatment of acute ischemic stroke, in particular after intra-arterial injection, in humans

In this example the tolerance and performance/efficiency of OTR4132-MD for the treatment of acute ischemic stroke (AIS) and/or biological effect of acute ischemic stroke (AIS) were carried out in humans. In particular, a study of the evolution of stroke lesion volume was carried out. The efficacy of OTR4132-MD was determined on the basis of functional scores and MRI volumetric analyses.

The biocompatible polymer used in the experiment is OTR4132, also designated OTR4132-MD when diluted in sterile saline solution (0.9%), is represented in figure 2 and mentioned in table 3 below :

| | | | | | |
|---|---|---|---|---|---|
| polymer | | A: glucose | X -CH₂COO⁻ | Y -SO₃⁻ | Z -OCCH₃ |

| Name of the RGTA | Average molecular weight M or mass +/-15% (MW in Dalton) | Dextran starting polymer (MW in Dalton) | % substitution CM (X)/glucose | % substitution -SO₃⁻ (Y)/glucose) | % substitution OCCH₃ (Z)/glucose |
|---|---|---|---|---|---|
| CMDSA OTR4132 | 220 000 | 110 000 | 60+/-20 | 140+/-20 | 20+/-5 |

The composition used, OTR4132-MD, comprises OTR4132 diluted in sterile saline solution (0.9%). The composition was provided as a sterile injectable medical device and presented in a 10 ml vial of sterile solution. Unique doses of OTR4132 at: 0.2 mg; 0.5 mg, 1 mg; 1.5 mg; 2 mg using compositions comprising concentrations of OTR4132-MD: 20 µg/mL; 50 µg/mL; 100 µg/mL; 150 µg/mL or 200 µg/mL

Treatment with OTR4132 was performed at 5 escalating doses ranging from 0.2 to 2 mg.

OTR4132-MD was used in anterior circulation acute ischemic stroke (AIS) patients re-vascularized (TICI score 2b-3) by endovascular thrombectomy combined or not with thrombolysis.

The administration was carried out by intra-arterial infusion at a rate of 1mL/min over 10 minutes, following endovascular thrombectomy recanalization (TICI score 2b - 3).

The study/follow-up was on 90 ± 14days.

The eligible patients for this study were included if all of the following conditions were met:
- Age between 45 and 80 years,
- Acute ischemic stroke in anterior circulation territory, identified by magnetic resonance imaging (MRI)
- Occlusion of anterior circulation i.e. internal carotid artery (ICA) or proximal middle cerebral artery (MCA) (M1 and/or M2 segment)
- Volume of the infarcted lesion estimated below two thirds of the MCA territory (diffusion MRI sequence)
- Endovascular thrombectomy initiated within 6 hours of stroke onset with known stroke onset time or Endovascular thrombectomy initiated within 6 to 16 hours of symptoms onset (last know well in case of unwitnessed onset) with a mismatch on brain MRI defined in *.
- Recanalization confirmed by angiography after endovascular treatment: TICI grade 2b - 3
- NIHSS at pre-screening (National Institute of Health Stroke Scale/Score), including hand testing: between 11 and 25
- No significant pre-stroke disability (pre-stroke modified Rankin Score (mRS): 0-1
- Able to follow neuro-rehabilitation program, and
- Patient or legally authorized representative (family member or trusted person if patient unable to give consent) or independent physician (if patient unable to give consent and if an authorized representative cannot be reached) has signed informed consent).
   * Perfusion core/penumbra mismatch:
      ► Infarct core volume <70 mL, and
      ► critically hypoperfused volume/infarct core volume >1.8, and
      ► mismatch volume >15mL

To the contrary, patients were not included if any of the following exclusion criteria are present:
- Previous symptomatic stroke,
- Pre-existing medical, neurological, or psychiatric disease that would confound the neurological evaluation,
- Contra-indication to MRI,
- Evidence of intracranial hemorrhage (ICH),
- At the discretion of the investigator, patients with co-morbidities associated with a life expectancy of less than 3 months or co-morbidities that could influence the study results or would complicate assessment of outcomes (e.g. dementia, psychiatric disease) or would make clinical follow-up difficult,
- History of allergy or anaphylactic reactions to any of the ingredients of OTR4132-MD or heparinoids,
- History of Hypersensitivity or anaphylactic reactions to iodinated contrast media,
- Severe renal failure with glomerular filtration rate (GFR) < 30 mL/min,
- Severe uncontrolled arterial hypertension e.g. systolic blood pressure > 185 mmHg or diastolic blood pressure > 110 mmHg, or intravenous medication necessary to reduce blood pressure,
- Increased risk of hemorrhage (such as medical history of significant bleeding disorders, major surgery or significant trauma in the past 3 months, any history of central nervous damage or suspected intracranial hemorrhage, symptoms suggestive of subarachnoid hemorrhage, even if the MRI is normal, international normalized ratio (INR)>1.3 or activated partial thromboplastin time (aPTT)>ULN (upper limit of normal),
- Suspected cerebral vasculitis based on medical history and imaging,
- Occlusions in multiple vascular territories,
- Evidence of intracranial tumor,
- Evidence of any prior intracranial intervention (i.e. neurosurgery, endovascular intervention) ,
- Worsening of medical or neurological conditions or per-procedures complications,
- Any other serious, advanced, or terminal illness (investigator judgment),
- Pregnant or breastfeeding or women without an adequate contraceptive method,
- Current participation in another investigation drug or device study, and/or
- The patient is not a member or beneficiary of the French social security system

The primary judgement criterion was the The rate of device-related severe adverse events occurring from baseline to 7 ± 2 days after inclusion.

In addition, the following safety parameters were collected :
- Survival rates (Time Frame: 24 ± 6 h, 7 ± 2 days, 30 ± 7 days, 90 ± 14 days after inclusion)
- All-cause death (Time Frame: 24±6h, 7 ± 2 days, 30 ± 7 days, 90 ± 14 days after inclusion)
- Stroke related death (Time Frame: 24 ± 6 h, 7 ± 2 days, 30 ± 7 days, 90 ± 14 days after inclusion)
- The rate of device related adverse events (AEs) at 24 ± 6 h, 7 ± 2 days, 30 ± 7days and 90 ± 14 days
- The rate of device related severe adverse events (SAE) at 24 ± 6h, 7 ± 2 days, 30±7days and 90 ± 14 days
- The rate of procedure related adverse events (AEs) at 24±6h, 7 ± 2 days, 30 ± 7 days, and 90 ± 14 days
- The rate of procedure related severe adverse events (SAE) at 24 ± 6 h, 7 ± 2 days, 30 ± 7 days, and 90 ± 14 days
- The rate of all adverse events (AEs) at 24 ± 6 h, 7 ± 2 days, 30 ± 7 days, and 90 ± 14 days
- The rate of all severe adverse events (SAE) at 24 ± 6 h, 7 ± 2 days, 30 ± 7 days, and 90 ± 14 days
- The rate of intracranial hemorrhage on 24-hour follow-up imaging (Time Frame: 24 ± 6 h) per ECASS III definition and per Heidelberg bleeding classification.

Sixteen patients were recruited in the study, during the study, OTR4132-MD has been supplied in five concentrations which was referred to as OTR4132-MD20, OTR4132-MD50, OTR4132-MD100, OTR4132-MD150, OTR4132-MD200 of OTR4132 concentrated at 20 µg/mL, 50 µg/mL, 100 µg/mL, 150 µg/mL, 200 µg/mL, respectively corresponding to total doses of 0.2 mg, 0.5 mg, 1 mg, 1.5 mg and 2 mg respectively. Three patients received the first dose of 0.2 mg, 3 patients received the second dose of 0.5 mg, 3 patients received the third dose of 1 mg, 4 patients received the fourth dose of 1.5 mg and 3 patients received the last dose of 2 mg.

Table 4 below represent the Demographic characteristics and main clinical results of the patients. In particular, in this table the stoke time, i.e. the time since the stroke, the occlusion type, i.e. the cerebral region were the occlusion appears, the stroke territory are mentioned.

**Table 4 : Demographic characteristics and main clinical results of the patients**

| **SUBJID** | **AGE** | **SEX** | **CONCEN TRATION OTR4132** | **STROKE TIME** | **OCCLUSION TYPE** | **STROKE TERRITORY** | **THROMB OLYSIS** | **DELAY FOR THROMBO LYSIS** | **DELAY FOR THROMBE CTOMY** | **TICI** |
|---|---|---|---|---|---|---|---|---|---|---|
| 001 | 76 | M | 20 | 11:00 | Proximal Middle Cerebral | Deep+SuperfM CA | Yes | 02:52:00 | 03:25:00 | 2b |
| 002 | 77 | F | 20 | 02:45 | Proximal Middle Cerebral | Deep+SuperfM CA | Yes | 03:45:00 | 04:35:00 | 2b |
| 003 | 59 | M | 20 | 08:00 | Internal Carotid | Deep+SuperfM CA | Yes | 02:20:00 | 03:06:00 | 3 |
| 004 | 53 | F | 50 | 08:00 | Proximal Middle Cerebral | Deep+SuperfM CA | No | | 06:28:00 | 3 |
| 005 | 71 | F | 50 | 12:00 | Proximal Middle Cerebral | Deep MCA | Yes | 03:40:00 | 05:30:00 | 3 |
| 006 | 69 | M | 50 | 23:30 | Proximal Middle Cerebral | Deep MCA | No | | 02:55:00 | 2b |
| 007 | 67 | M | 100 | 22:00 | Internal Carotid | Deep+SuperfM CA | Yes | 08:15:00 | 13:03:00 | 3 |
| 008 | 64 | M | 100 | 16:50:00 | Proximal Middle Cerebral | Deep+SuperfM CA | No | | 03:21:00 | 2b |
| 009 | 71 | F | 100 | 01:00 | Proximal Middle Cerebral | Deep MCA | No | | 15:18:00 | 2b |
| 010 | 57 | M | 150 | 05:00 | Internal Carotid | Superficial MCA | Yes | No data | 04:55:00 | 3 |
| 011 | 80 | M | 150 | 00:30 | Proximal Middle Cerebral | Deep+SuperfM CA | Yes | 01:30:00 | 05:00:00 | 3 |
| 012 | 77 | M | 150 | 07:30 | Internal Carotid | Deep+SuperfM CA | Yes | 02:40:00 | 03:58:00 | 2b |
| 013 | 42 | F | 150 | 08:15 | Proximal Middle Cerebral | Deep+SuperfM CA | Yes | 03:45:00 | 03:45:00 | 2b |
| 014 | 61 | M | 200 | 09:30 | Proximal Middle Cerebral | | Yes | 01:30:00 | 05:00:00 | 2b |
| 015 | 69 | M | 200 | 04:00 | Internal Carotid | Deep MCA | Yes | 05:20:00 | 05:44:00 | 3 |
| 016 | 56 | F | 200 | 07:45 | Proximal Middle Cerebral | Deep+SuperfM CA | Yes | 02:37:00 | 03:50:00 | 3 |

| | | | | | | | | | | |
|---|---|---|---|---|---|---|---|---|---|---|
| Legend: M: male, F: female, MCA: middle cerebral artery, SubID: subject number in the study, TICI= thrombolysis and cerebral infarction reperfusion categories. | | | | | | | | | | |

For all the patients, the following efficacy parameters were also measured:
The NIH stroke scale (NIHSS) and the mRS (modified Rankin Scale).

The NIH stroke scale (https://www.ninds.nih.gov/health-information/public-education/know-stroke/health-professionals/nih-stroke-scale) is based on the collection of 15 clinical neurological items. It allows for an accurate and rapid assessment of observed deficits by the clinical investigator. A large number of publications have shown that the NIHSS score at 24 hours is the best prognostic factor for long-term functional disability and is closely correlated with disability scores at 3 months. An NIHSS score between 1 and 4 means a minor stroke, between 5 and 15, a moderate stroke, between 15 and 20, severe, and above 20 points, a severe stroke. The maximum score is 42.

The mRS (modified Rankin Scale),( Modified Rankin Scale (MRS) Evidence Reviewed as of before: 19-08-2008 Author(s)*: Lisa Zeltzer, MSc OT Editor(s): Nicol Korner-Bitensky, PhD OT; Elissa Sitcoff, BA BSc; Sabrina Figueiredo, BSc was determined by the clinical investigator. This well-known score is based on 6 categories: "0" means No symptoms, "1" means no disability apart from symptoms: activities and autonomy maintained, "2" means low disability: unable to perform usual activities but independent, "3" means moderate disability: needs help but walks without assistance, "4" means moderately severe disability: walking and daily movements impossible without assistance and "5" means major disability: permanent bed rest, incontinence and permanent nursing care.

The NIHSS (NIH stroke scale), mRS (modified Rankin Scale), were measured at different times after administration of OTR4132: V1: 24 hours, V2: 7 days, V3: 1 month and/or V4: 3 months. The obtained results are represented in table 5 below.

**Table 5 : results of NIHSS and mRS:**

| **SUB** | **Dose** | **NIHSS Scr** | **NIHSS V1** | **NIHSS V2** | **NIHSS V3** | **NIHSS V4** | **mRS Scr** | **mRS V1** | **mRS V2** | **mRS V3** | **mRS V4** |
|---|---|---|---|---|---|---|---|---|---|---|---|
| 001 | 0,2 mg | 11 | 5 | 0 | 0 | 0 | 4 | 4 | 1 | 0 | 0 |
| 002 | 0,2 mg | 19 | 8 | 3 | NA | 11 | 4 | 4 | 3 | 3 | 5 |
| 003 | 0,2 mg | 22 | 11 | 6 | 6 | 4 | 5 | 4 | 3 | 3 | 2 |
| 004 | 0,5 mg | 19 | 15 | 15 | NA | NA | 5 | 4 | 4 | 4 | NA |
| 005 | 0,5 mg | 15 | 2 | 0 | NA | 0 | 5 | 4 | 1 | 1 | 1 |
| 006 | 0,5 mg | 15 | 3 | 1 | 2 | 1 | 4 | NA | 1 | 1 | 1 |
| 007 | 1 mg | 19 | 13 | 5 | NA | 3 | 5 | 4 | 1 | 2 | 2 |
| 008 | 1 mg | 5* | 8 | 7 | NA | 3 | 3 | 4 | 4 | 3 | 1 |
| 009 | 1 mg | 17 | 14 | 11 | 11 | 12 | 4 | 5 | 5 | 5 | 4 |
| 010 | 1,5 mg | 15 | 3 | 0 | NA | 1 | 5 | 3 | 0 | 2 | 2 |
| 011 | 1,5 mg | 17 | 6 | 6 | NA | NA | 5 | NA | 4 | NA | NA |
| 012 | 1,5 mg | 14 | 18 | 15 | 15 | 15 | 5 | 5 | 5 | 4 | 4 |
| 013 | 1,5 mg | 11 | 3 | 0 | 0 | 0 | 4 | NA | 0 | 1 | 0 |
| 014 | 2 mg | 18 | 1 | 0 | 0 | 0 | 5 | NA | 0 | 2 | 1 |
| 015 | 2 mg | 14 | 0 | 0 | 0 | 0 | 4 | 4 | 1 | 1 | 1 |
| 016 | 2 mg | 11 | 0 | 0 | 0 | 0 | 5 | 1 | 3 | 3 | 1 |

No serious adverse event attributable to the treatment was noticed at any of the 5 tested doses.

Figure 8 represents the evolution of the mean NIH Stroke Scale (NIHSS) over time.

As shown in the table 5 above and on figure 8, surprisingly, the NIH Stroke Scale (NIHSS) observed with the first 4 doses (from 0.2 to 1.5 mg) does not reveal a significative evolution of the score within 24 hours after stroke. In these four dose groups the mean percentage of improvement of the NIHSS score ranged from +28.6% to -80% with an average of -44.8%. Surprisingly, a complete clinical recovery was observed within 24 hours after stroke with a dose of 2 mg. Among the 3 patients treated with this dose, 2 recovered by 100% at 24 hours and one at 94.4%.

In addition, as shown in the table 5 above and on figure 8, surprisingly, the NIH Stroke Scale (NIHSS) reveals a significative evolution of the score V4: 3 months after stroke showing a full recovery of functional abilities.

Figure 9 represents the mean of Modified RANKIN score (mRS) over time.

As demonstrated in table 5 and in figure 9, the treatment at higher dose (2 mg) allows clearly to reduce the value of the Modified RANKIN score compared to lower doses. The effect is especially visible at day 1.

Volumetric analyses of stroke lesion volumes of the patients were also assessed from brain MRIs. Stroke lesion volume was assessed using DICOM viewer software image segmentation and analysis tools. Baseline measurements involved capturing ADC (Apparent Diffusion Coefficient) values using the Rapid software (iSchemaView, Menlo Park, CA), with manual corrections performed in any measurements that were identified as artifacts by clinicians. In addition, the subacute FLAIR lesion was manually outlined with precision drawing tools, ensuring accurate demarcation of the lesion boundaries. Measurement tools were then utilized to calculate the total volume of these outlined regions. The software's ability to handle multiplanar reconstructions and cross-sectional area calculations facilitated a comprehensive and assessment of the total stroke lesion volume. Regions of hemorrhagic transformation, if present, were included in the total stroke lesion volume assessment.

The stroke volumes of the patients were determined at V1: 24 hours, V2: 7 days, V3: 1 month and/or V4: 3 months.

Figure 10 represents the median percentage of volume reduction between visit 1 (24 hours) and visit 4 (3 months) according to the total dose of treatment. As shown on this figure, a decrease in lesion volume was observed at each dose. Surprisingly, the obtained results clearly show that the volume reduction increases as doses increase. This indicates and effect of treatment at higher doses.

A study of the effects of dose per lesion volume unit was also carried out. In particular the change in MRI lesion volume at 3 months was expressed as a function of the dose administered per initial stroke lesion volume (mg/ml). This dose/volume was calculated by dividing the total dose administered (µg) by the "initial" stroke volume (ml) defined as the highest volume measured within 24 hours after hospital admission.

Figure 11 represents the stroke lesion volume change in each patient expressed as the percentage of volume reduction between initial stroke lesion volume (24 hours) and visit 4 (3 months) as a function of the dose administered per initial stroke lesion volume (µg/ml). As demonstrated on figure 8, by looking at the stroke lesion volume change at 3 months, patients who received less than 10 µg/ml had less lesion reduction at 3 months (median= -38.2%) compared to those who received more than 10 µg/ml (median= -81.8%). The results were compared using the Mann-Whitney test. The comparison of these results was statistically significant (p=0,00067, Mann-Whitney).

### A Study of the mRS score at 3 months regarding the total dose administered/initial lesion volume was carried out.

Figure 12 represents the obtained mRS scores at 3 months as a function of total dose administered/ initial stroke lesion volume. As demonstrated on this figure, the obtained results clearly demonstrate that the MRI results correlate well with the mRS score at 3 months: patients having received less than 10 µg/ml of lesion volume have higher mRS compared to those who received more than 10 µg/ml of lesion volume (p=0,0089, Mann-Whitney).

A study of hemorrhagic transformations was carried out based on MRIs at 24 hours. Analyses were performed by an independent skilled neuroradiologist based on V1 brain MRIs. Hemorrhagic transformations were classified by the expert using the Heidelberg Bleeding Classification from less severe to more severe hemorrhages: NO: absence of intracranial hemorrhage; 1a: scattered small petechiae, no mass effect; 1b: confluent petechiae, no mass effect; 2: intracerebral hemorrhage within and beyond infarcted brain tissue; 3b: intraventricular hemorrhage; 3d: subdural hemorrhage.

Figure 13 is a CIRCOS representation for contingency data that correlates the total doses received (from 0.2 to 2 mg: 0.2, 0.5, 1.0, 1.5, or 2.0 mg of OTR4132) with the degree of hemorrhagic transformation according to Heidelberg Bleeding Classification (from 0 to IIId). As shown in figure 12, the dose of 1 mg clearly separates the higher doses (1.5 and 2 mg) which are associated with less severe hemorrhagic transformations (0 to Ib) from lower doses (0.2 and 0.5 mg) which are associated with more severe hemorrhagic transformations (Ib to IIId). These results indicate a positive effect of OTR4132 administration at higher dose on the prevention of hemorrhagic transformation.

Overall, the obtained results clearly demonstrate that the use of a biopolymer according to the invention, for example OTR4132, allows to treat ischemic stroke.

The results demonstrate that the use of OTR4132 for the treatment of stroke allows surprisingly and unexpectedly, to reduce the stroke lesion volume .

The results also clearly demonstrate that the use of OTR4132 for the treatment of ischemic stroke allows surprisingly and unexpectedly, to improve many aspects of neurological functional recovery after stroke.

The results also clearly demonstrate that the use of OTR4132 for the treatment of ischemic stroke allows surprisingly and unexpectedly, to decrease the risk of hemorrhagic transformation at 24 hours after reperfusion.

As demonstrated above, the in vivo experiments clearly demonstrate that administration of OTR4132-MD intra-arterially provides surprising therapeutical effects.

The results also demonstrate that the use of OTR4132 for the treatment of ischemic stroke, for example via intra-arterial administration, is effective above a specific dose which corresponds to at least 10 µg/ml of initial stroke lesion volume. The corresponding total efficient dose in humans is at least 1 mg, preferably 2 mg, but depending on the size of the stroke lesion volume, any dose administered intra-arterially allowing to reach the threshold of 10 µg/ml of initial stroke lesion volume allows the treatment of the ischemic stroke.

The results also demonstrate that the intravenous administration of the biopolymer OTR4131 (HSm4131) for the treatment of ischemic stroke, is effective above a specific therapeutic dose which corresponds to at least 200 µg/ml of initial stroke lesion volume. The corresponding total efficient dose in humans should be at least 20 mg but depending on the size of the initial infarct, any dose administered intra-venously allowing to reach the threshold of 200 µg/ml of initial stroke lesion volume allows the treatment of ischemic stroke.

### List of references

1. Arba F, Rinaldi C, Caimano D, Vit F, Busto G, Fainardi E. Blood-Brain Barrier Disruption and Hemorrhagic Transformation in Acute Ischemic Stroke: Systematic Review and Meta-Analysis. Front Neurol. 2021 Jan 21;11-594613. doi: 10.3389/fneur.2020.594613. PMID: 33551955; PMCID: PMC7859439.
2. Benjamin, E. J., Virani, S. S., Callaway, C. W., Chamberlain, A. M., Chang, A. R., Cheng, S., et al. (2018). Heart Disease and Stroke Statistics-2018 Update: A Report From the American Heart Association. Circulation, 137(12), e67-e492. doi:10.1161/CIR.0000000000000558
3. Campbell, B. C., Donnan, G. A., Mitchell, P. J., & Davis, S. M. (2016). Endovascular thrombectomy for stroke: current best practice and future goals. Stroke Vasc Neurol, 1(1), 16-22. doi:10.1136/svn-2015-000004
4. Chuquet J, Benchenane K, Toutain J, MacKenzie E, Roussel S and Touzani O, Selective Blockade of Endothelin-B Receptors Exacerbates Ischemic Brain Damage in the Rat, Originally published31 Oct 2002 https://doi.org/10.1161/01/01.STR.0000039401.48915.9F
5. Coupland AP, Thapar A, Qureshi MI, Jenkins H, Davies AH. The definition of stroke. J R Soc Med. 2017 Jan;110(1):9-12. doi: 10.1177/0141076816680121. Epub 2017 Jan 13. PMID: 28084167; PMCID: PMC5298424.
6. Cramer, S. C., & Riley, J. D. (2008). Neuroplasticity and brain repair after stroke. Curr Opin Neurol, 21(1), 76-82. doi:10.1097/WCO.0b013e3282f36cb6
7. Cramer SC. Drugs to Enhance Motor Recovery After Stroke. Stroke. 2015 Oct;46(10):2998-3005. doi: 10.1161/STROKEAHA.115.007433. Epub 2015 Aug 11. PMID: 26265126; PMCID: PMC4589468.
8. Dzyubenko, E., Manrique-Castano, D., Kleinschnitz, C., Faissner, A., & Hermann, D. M. (2018). Role of immune responses for extracellular matrix remodeling in the ischemic brain. Ther Adv Neurol Disord, 11, 1756286418818092. doi:10.1177/1756286418818092
9. Feigin, V. L., Forouzanfar, M. H., Krishnamurthi, R., Mensah, G. A., Connor, M., Bennett, D. A., et al. (2014). Global and regional burden of stroke during 1990-2010: findings from the Global Burden of Disease Study 2010. Lancet, 383(9913), 245-254. doi:10.1016/s0140-6736(13)61953-4
10. Goyal, M., Menon, B. K., van Zwam, W. H., Dippel, D. W., Mitchell, P. J., Demchuk, A. M., et al. (2016). Endovascular thrombectomy after large-vessel ischaemic stroke: a meta-analysis of individual patient data from five randomised trials. Lancet, 387(10029), 1723-1731. doi:10.1016/S0140-6736(16)00163-X
11. Guo, X., & Miao, Z. (2021). Advances in mechanical thrombectomy for acute ischaemic stroke from large vessel occlusions. Stroke and vascular neurology, svn-2021-000972. Advance online publication. doi:10.1136/svn-2021-000972
12. Gutiérrez-Fernández M, Fuentes B, Rodríguez-Frutos B, Ramos-Cejudo J, Vallejo-Cremades MT, Díez-Tejedor E. Trophic factors and cell therapy to stimulate brain repair after ischaemic stroke. J Cell Mol Med. 2012 Oct;16(10):2280-90. doi: 10.1111/j. 1582-4934.2012.01575.x. PMID: 22452968; PMCID: PMC3823421.
13. Harpaz D, Seet RCS, Marks RS, Tok AIY. Blood-Based Biomarkers Are Associated with Different Ischemic Stroke Mechanisms and Enable Rapid Classification between Cardioembolic and Atherosclerosis Etiologies. Diagnostics (Basel). 2020 Oct 9;10(10):804. doi: 10.3390/diagnostics10100804. PMID: 33050269; PMCID: PMC7600601.
14. Matei N, Camara J, Zhang JH. The Next Step in the Treatment of Stroke. Front Neurol. 2021 Jan 22;11:582605. doi: 10.3389/fneur.2020.582605. PMID: 33551950; PMCID: PMC7862333.
15. Quittet M. S., Touzani O., Sindji L., Cayon J., Fillesoye F., Toutain J., et al. (2015). Effects of mesenchymal stem cell therapy, in association with pharmacologically active microcarriers releasing VEGF, in an ischaemic stroke model in the rat. Acta Biomater. 15 77-88. 10.1016/j.actbio.2014.12.017
16. Ospel JM, Hill MD, Menon BK, Demchuk A, McTaggart R, Nogueira R, Poppe A, Haussen D, Qiu W, Mayank A, Almekhlafi M, Zerna C, Joshi M, Jayaraman M, Roy D, Rempel J, Buck B, Tymianski M, Goyal M; ESCAPE-NA1 investigators. Strength of Association between Infarct Volume and Clinical Outcome Depends on the Magnitude of Infarct Size: Results from the ESCAPE-NA1 Trial. AJNR Am J Neuroradiol. 2021 Aug;42(8):1375-1379. doi: 10.3174/ajnr.A7183. Epub 2021 Jun 24. PMID: 34167959; PMCID: PMC8367613
17.Shi, L., Rocha, M., Leak, R. K., Zhao, J., Bhatia, T. N., Mu, H., et al. (2018). A new era for stroke therapy: Integrating neurovascular protection with optimal reperfusion. Journal of cerebral blood flow and metabolism : official journal of the International Society of Cerebral Blood Flow and Metabolism, 38(12), 2073-2091. doi:10.1177/0271678X18798162
18. The Stroke Association. (2018). State of the Nation Stroke Statistics, 5. Retrieved from www.stroke.org.uk/system/files/sotn_2018.pdf website:
19. T. Gerriets, E. Stolz, M. Walberer, C. Müller, A. Kluge, A. Bachmann, M. Fisher, M. Kaps, G. Bachmann, Noninvasive quantification of brain edema and the space-occupying effect in rat stroke models using magnetic resonance imaging, Stroke, 35 (2) (2004), pp. 566-571
20. YASUNORI I et al. Procede de sulfonation de composes comprenant des groupements hydroxyles (oh) libres ou des amines primaires ou secondaires. Biomaterials, 2011, vol. 32, 769-776
21. PETIT E. et al. Biomacromolecules, Mars 2004, vol.5 (2), 445-52
22. US7396923

## Claims

1. A pharmaceutical composition for use in the treatment of ischemic stroke, said composition comprising
- a biocompatible polymer of the following general formula (I)
AaXxYy (I)
wherein:
A is a monomer,
X is an R₁COOR₂ or -R₉(C=O)R₁₀ group,
Y is an O or N-sulfonate group and has one of the following formulas -R₃OSO₃R₄, -R₅NSO₃R₆, R₇SO₃R₈ wherein:
R₁, R₃, R₅ and R₉ are independently an aliphatic hydrocarbon chain, optionally branched and/or unsaturated and optionally containing one or more aromatic rings with the exception of benzylamine and benzylamine sulfonate, R₂, R₄, R₆ and R₈ are independently a hydrogen atom or an M⁺ cation,
R₇ and R₁₀ are independently a bond, an aliphatic hydrocarbon chain, optionally branched and/or unsaturated,
a is the number of monomers,
x is the rate of substitution of the A monomers by X groups,
y is the rate of substitution of the A monomers by Y groups
wherein said biocompatible polymer is administered in the treatment of ischemic stroke at a dose from 0.5 to 5 mg per day.

2. A pharmaceutical composition for use according to claim 1, wherein said pharmaceutical composition is for intra-arterial administration and the dose of biocompatible polymer is of at least 10 µg per ml⁻¹ by weight of biocompatible polymer with respect to the total initial stroke lesion volume.

3. A pharmaceutical composition for use according to claim 1, wherein said pharmaceutical composition is for intra-venous administration, and the dose of biocompatible polymer is of at least 100 µg.mL⁻¹, preferably 200 µg.mL⁻¹ by weight of biocompatible polymer with respect to the total initial stroke lesion volume.

4. The composition for use according to any one of claims 1 to 3, wherein the identical or different A monomers are selected from sugars, esters, alcohols, amino acids, nucleotides, nucleic acids, proteins or derivatives thereof.

5. The composition for use according to any one of claims 1 to 4, wherein the number of monomers "a" is such that the weight of said polymers of formula (I) is greater than or equal to 2000 Daltons.

6. The composition for use according to any one of claims 1 to 5, wherein the rate of substitution "x" is between 10 and 150%.

7. The composition for use according to any one of the preceding claims, wherein the rate of substitution "y" is between 10 and 170%.

8. The composition for use according to any one of the preceding claims, wherein said biocompatible polymer further comprises functional chemical groups Z, different from X and Y, capable of conferring to said polymer additional biological or physicochemical properties.

9. The composition for use according to claim 8, wherein the rate of substitution "z" of all the A monomers by Z groups is between 1 and 50%.

10. The composition for use according to claim 8 or 9, wherein the Z group is a substance capable of conferring to said polymers an improved solubility or lipophilicity.

11. The composition for use according to claim 10, wherein the Z groups are identical or different and are selected from the group consisting of amino acids, fatty acids, fatty alcohols, ceramides, or derivatives thereof, or nucleotide addressing sequences.

12. The composition for use according to any one of claims 1 to 11, wherein the polymer concentration is from 0.01 to 3000 µg/mL by weight of biocompatible polymer with respect to the volume of the composition.

13. The composition for use according to any of the preceding claims wherein the composition is used in combination with at least one other active ingredient, particularly another therapeutically active ingredient or therapeutic agent, selected from the group comprising fibrinolytic, anticoagulant, anti-aggregant, neuroprotective drugs and combinations thereof.

14. The composition for use according to any of the preceding claims wherein the treatment of ischemic stroke further allows the treatment of intracerebral hemorrhage.

15. The composition for use according to claim 14 wherein the treatment of ischemic stroke further allows to prevent and/or reduce hemorrhagic transformation due to reperfusion after ischemic stroke.
